(19) Europäisches Patentamt
European Patent Office
Office européen des brevets

(11) Veröffentlichungsnummer : **0 386 561 B1**

(12) # EUROPÄISCHE PATENTSCHRIFT

(45) Veröffentlichungstag der Patentschrift : **21.04.93 Patentblatt 93/16**

(51) Int. Cl.$^5$ : **C07C 251/38,** A01N 37/50

(21) Anmeldenummer : **90103628.5**

(22) Anmeldetag : **24.02.90**

(54) **Substituierte Oximether und Fungizide, die diese Verbindungen enthalten.**

(30) Priorität : **09.03.89 DE 3907629**
**10.11.89 DE 3937457**

(43) Veröffentlichungstag der Anmeldung : **12.09.90 Patentblatt 90/37**

(45) Bekanntmachung des Hinweises auf die Patenterteilung : **21.04.93 Patentblatt 93/16**

(84) Benannte Vertragsstaaten : **AT BE CH DE DK ES FR GB GR IT LI NL SE**

(56) Entgegenhaltungen :
**EP-A- 0 203 608**
**EP-A- 0 242 081**
**EP-A- 0 253 213**
**EP-A- 0 254 426**
**EP-A- 0 260 794**
**EP-A- 0 278 595**
**EP-A- 0 307 103**
**GB-A- 2 193 495**

(73) Patentinhaber : **BASF Aktiengesellschaft**
**Carl-Bosch-Strasse 38**
**W-6700 Ludwigshafen (DE)**

(72) Erfinder : **Schuetz, Franz, Dr.**
**Budapester Strasse 45**
**W-6700 Ludwigshafen (DE)**
Erfinder : **Sauter, Hubert, Dr.**
**Neckarpromenade 20**
**W-6800 Mannheim 1 (DE)**
Erfinder : **Harreus, Albrecht, Dr.**
**Teichgasse 13**
**W-6700 Ludwigshafen (DE)**
Erfinder : **Rohr, Wolfgang, Dr.**
**In der Dreispitz 13**
**W-6706 Wachenheim (DE)**
Erfinder : **Hepp, Michael, Dr.**
**Im Steg 38**
**W-6802 Ladenburg (DE)**
Erfinder : **Brand, Siegbert, Dr.**
**Hegelstrasse 39**
**W-6940 Weinheim (DE)**
Erfinder : **Wenderoth, Bernd, Dr.**
**Schwalbenstrasse 26**
**W-6840 Lampertheim (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

**EP 0 386 561 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Oximether und Fungizide, welche diese Verbindungen enthalten.

Es ist bekannt, Oximether wie zum Beispiel das 2-(Phenoxymethyl)-phenylglyoxylsäuremethylester-O-methyloxim als Fungizide zu verwenden (EP 253 213). Ihre fungizide Wirkung ist jedoch oft nicht ausreichend.

Es wurde nun gefunden, daß substituierte Oximether der allgemeinen Formel I,

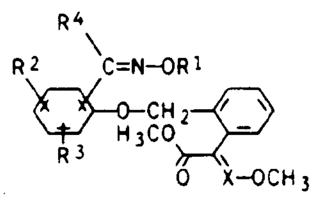

I

in der

$R^1$

$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist:

$C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,

$R^2$ und $R^3$

gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,

$R^4$

Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und

X CH oder N bedeutet, eine ausgezeichnete fungizide Wirkung besitzen, die besser ist als die der bekannten Oximether.

Die in der allgemeinen Formel aufgeführten Reste können beispielsweise folgende Bedeutung haben:

$R^1$

kann z.B. $C_1$-$C_6$-Alkyl ($C_1$-$C_4$-Alkyl) (z.B. Methyl, Ethyl, n- oder iso-Propyl, n-, iso-, sec.- oder tert.-Butyl, n-, iso-, sec.-tert.- oder neo-Pentyl, Hexyl), $C_3$-$C_6$-Alkenyl (z.B. Allyl, 2-Butenyl, 3-Butenyl, 1-Methyl-2-propenyl, 2-Methyl-2-propenyl), $C_3$-$C_4$-Alkinyl (z.B. Propargyl, 2-Butinyl), $C_1$-$C_6$-Halogenalkyl, (z.B. 2-Fluorethyl), $C_3$-$C_6$-Halogenalkenyl (z.B. 3-Chlorallyl), $C_1$-$C_4$-Alkoxy-$C_1$-$C_6$-alkyl (z.B. 2-Methoxyethyl, 3-Ethoxypropyl), $C_3$-$C_6$-Cycloalkyl (z.B. Cyclopropyl, Cyclobutyl, Cyclopentyl, Cyclohexyl), $C_3$-$C_6$-Cycloalkyl-$C_1$-$C_4$-alkyl (z.B. Cyclopropylmethyl, Cyclohexylmethyl), Cyan-$C_1$-$C_6$-alkyl (z.B. Cyanmethyl, 3-Cyanpropyl), $C_1$-$C_6$-Alkoxy-carbonyl-$C_1$-$C_6$-alkyl (z.B. Ethoxycarbonylmethyl, tert.-Butoxycarbonylmethyl, tert.Butoxycarbonylpropyl), Aryl-(Phenyl)-$C_1$-$C_6$-alkyl (z.B. Benzyl, 2-Phenylethyl, 3-Phenylpropyl, 4-Phenylbutyl), Heteroaryl-(Pyridyl, Thienyl)-$C_1$-$C_6$-alkyl (z.B. Pyrid-3-yl-methyl, Thien-2-yl-methyl), Aryl-(Phenyl)-$C_3$-$C_6$-alkenyl (z.B. 4-Phenyl-2-butenyl, 4-Phenyl-3-butenyl), Aryloxy-(Phenoxy)-$C_1$-$C_6$-alkyl (z.B. Phenoxymethyl, Phenoxyethyl, Phenoxypropyl, Phenoxybutyl, Naphthoxymethyl, Naphthoxyethyl) sein,

wobei der aromatische (Phenyl) oder heteroaromatische (Pyridyl, Thienyl) Ring gegebenenfalls durch einen oder mehrere z.B. 1 bis 5, insbesondere 1 bis 3 der folgenden Reste substituiert ist:

$C_1$-$C_4$-Alkyl (z.B. Methyl, Ethyl, Propyl, Butyl), $C_1$-$C_2$-Halogenalkyl, (z.B. Trifluormethyl, Trichlormethyl), $C_3$-$C_6$-Cycloalkyl (z.B. Cyclopropyl, Cyclobutyl, Cyclopentyl, Cyclohexyl), $C_1$-$C_4$-Alkoxy (z.B. Methoxy, Ethoxy, Propoxy, Butoxy), $C_1$-$C_2$-Halogenalkoxy (z.B. Trifluormethoxy), Halogen (z.B. Fluor, Chlor, Brom), Aryl (z.B. Phenyl), Aryloxy (z.B. Phenoxy),

$R^2$ und $R^3$

können gleich oder verschieden sein und Wasserstoff, $C_1$-$C_4$-Alkyl (z.B. Methyl, Ethyl, n- oder iso-Propyl, Butyl), $C_1$-$C_2$-Halogenalkyl, (z.B. Trifluormethyl, Trichlormethyl), $C_1$-$C_4$-Alkoxy (z.B. Methoxy, Ethoxy, n-oder iso-Propoxy, Butoxy), $C_1$-$C_2$-Halogenalkoxy (z.B. Trifluormethoxy), Halogen (z.B. Fluor, Chlor, Brom, Jod), Cyano oder Nitro sein,

$R^4$

kann z.B. $C_1$-$C_6$-Alkyl, ($C_1$-$C_4$-Alkyl) (z.B. Methyl, Ethyl, n- oder iso-Propyl, n-, iso-, sec.- oder tert.-Butyl, n-, iso, sec.- tert. oder neo-Pentyl, Hexyl) oder Aryl (z.B. Phenyl) sein, wobei der aromatische Ring gegebenenfalls durch einen oder mehrere z.B. 1 bis 5, insbesondere 1 bis 3 der folgenden Reste substituiert ist: $C_1$-$C_4$-Alkyl (z.B. Methyl, Ethyl, Propyl, Butyl), $C_1$-$C_2$-Halogenalkyl (z.B. Trifluormethyl, Trichlormethyl), $C_1$-$C_4$-Alkoxy (z.B. Methoxy, Ethoxy, Propoxy, Butoxy), $C_1$-$C_2$-Halogenalkoxy (z.B. Difluormethoxy, Trifluormethoxy), Halogen (z.B. Fluor, Chlor, Brom, Jod) Cyano oder Nitro,

x kann CH oder N bedeuten.

Der Rest $-C(R^4)=N-O-R^1$ kann am Phenylrest im Hinblick auf den Rest $-O-CH_2$-in 2-, oder bevorzugt in 3- oder 4-Stellung stehen.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographie, in die einzelnen Komponenten getrennt werden. Sowohl die einzelnen isomeren Verbindungen als auch ihre Gemische werden von der Erfindung umfaßt und sind als Fungizide brauchbar. Bezüglich der Gruppierung $-C(COOCH_3)=X-OCH_3$ sind diejenigen Verbindungen bevorzugt, in denen die Gruppen $COOCH_3$ und $OCH_3$ an der C=X-Doppelbindung E-Konfiguration besitzen. Bezüglich der Gruppierung $-C(R^4)=N-OR^1$ sind diejenigen Verbindungen bevorzugt, in denen $R^4$ und $OR^1$ an der C=N-Doppelbindung Z-Konfiguration besitzen.

Die Darstellung der neuen Verbindungen der allgemeinen Formel I gemäß Anspruch 1 erfolgt beispielsweise so, daß man einen substituierten Oximether der allgemeinen Formel II mit einer substituierten Benzylverbindung der allgemeinen Formel III, in der Y eine Abgangsgruppe (z.B. Chlorid, Bromid, p-Toluolsulfonat, Methansufonat, Trifluormethansulfonat) bedeutet, umsetzt.

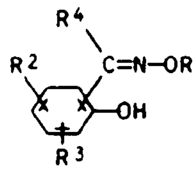

II

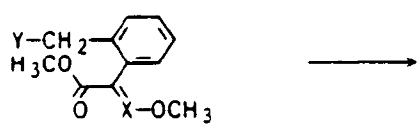

III

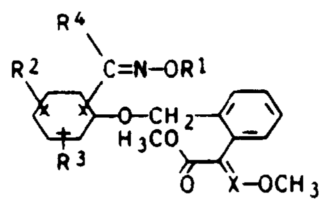

I

$R^1$, $R^2$, $R^3$, $R^4$ und X haben die oben genannten Bedeutungen.

Die beschriebenen Umsetzungen können z.B. in einem inerten Lösungs- oder Verdünnungsmittel (z.B. Aceton, Acetonitril, Dimethylsulfoxid, Dimethylformamid, N-Methylpyrrolidon, N,N'-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dixoheptyl)-amin zuzusetzen.

Alternativ kann auch so vorgegangen werden, daß man die Verbindungen der allgemeinen Formel II zunächst mit einer Base (z.B. Natriumhydroxid, Kaliumhydroxid, Natriummethanolat) in die entsprechenden Natrium- bzw. Kaliumphenolate überführt und diese dann in einem inerten Lösungs- oder Verdünnungsmittel (z.B. Dimethylformamid) mit den substituierten Benzylverbindungen der allgemeinen Formel III zu den erfindungsgemäßen Verbindungen der Formel I umsetzt.

Die entsprechenden Umsetzungen können auch im Zweiphasensystem (z.B. Tetrachlorkohlenstoff/Wasser) durchgeführt werden. Als Phasentransfer-Katalysatoren kommen z.B. Trioctylpropylammoniumchlorid oder Cetyltrimethylammoniumchlorid in Betracht.

Die zur Herstellung der neuen Verbindungen der allgemeinen Formel I benötigten substituierten Oximether der Formel II sind entweder bekannt oder können analog zu bekannten Verfahren hergestellt werden.

Zur Darstellung der erfindungsgemäßen Verbindungen der allgemeinen Formel I werden ferner die substituierten Benzylverbindungen der allgemeinen Formel III benötigt. Verbindungen der allgemeinen Formel IIIa (X=N, Y=Chlorid, Bromid) erhält man durch Halogenierung des 2-Methylphenylglyoxylsäure-methylester-O-methyloxims IV nach literaturbekannten Methoden. Dies erreicht man zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder durch Umsetzung mit N-Chlor- bzw. N-Bromsuccinimid (vgl. Horner, Winkelmann, Angew. Chem. 71 (1959) 349).

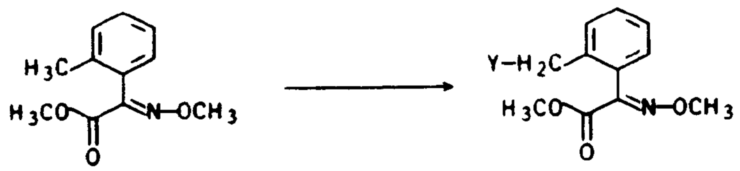

IV IIIa: Y = Chlorid, Bromid

2-Methyl-phenylglyoxylsäure-methylester-O-methyloxim IV läßt sich herstellen, indem man 2-Methyl-phenylglyoxylsäure-methylester V z.B. a) mit O-Methylhydroxylamin-hydrochlorid umsetzt oder b) mit Hydroxylaminhydrochlorid zum entsprechenden Oxim umsetzt und dieses mit einem Methylierungsmittel der Formel $CH_3$-L, in der L eine Abgangsgruppe (z.B. Chlorid, Bromid, Jodid, Methylsulfat) bedeutet, zur Reaktion bringt (vgl. DE-36 23 921).

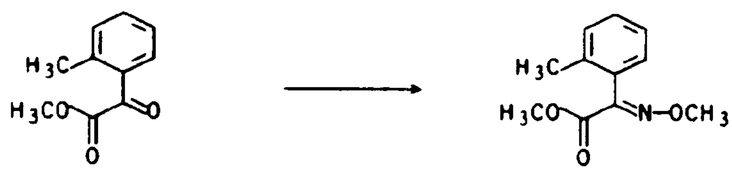

V IV

Benzylhalogenide der allgemeinen Formel IIIa (X=N, Y=Chlorid, Bromid) erhält man auch, wenn man 2-Halogenmethyl-phenylglyoxylsäuremethylester der Formel VI (Hal=Chlorid, Bromid) a) mit O-Methylhydroxylamin-hydrochlorid umsetzt oder b) mit Hydroxylaminhydrochlorid zum entsprechenden Oxim umsetzt und dieses mit einem Methylierungsmittel der Formel $CH_3$-L, in der L eine Abgangsgruppe (z.B. Chlorid, Bromid, Jodid, Methylsulfat) bedeutet, zur Umsetzung bringt (vgl. DE-36 23 921).

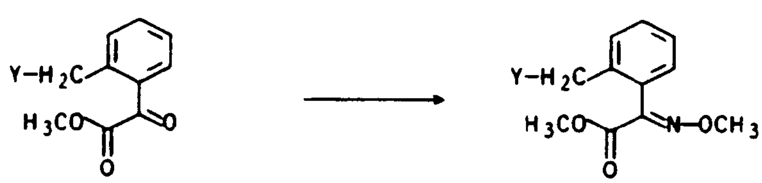

VI IIIa

Y = Chlorid, Bromid

2-Halogenmethyl-phenylglyoxylsäure-methylester der Formel VI (Y=Chlorid, Bromid) lassen sich herstellen, indem man 2-Methyl-phenylglyoxylsäuremethylester V nach literaturbekannten Methoden halogeniert. Die Umsetzung erfolgt zum Beispiel mit Brom oder Chlor in einem inerten Lösungsmittel (z.B. Tetrachlormethan), gegebenenfalls unter Belichtung mit einer Lichtquelle (z.B. Hg-Dampf-Lampe, 300 W) oder mit N-Chlor- bzw. N-Brom-Succinimid (vgl. Horner, Winkelmann, Angew. Chem. 71 (1959) 349).

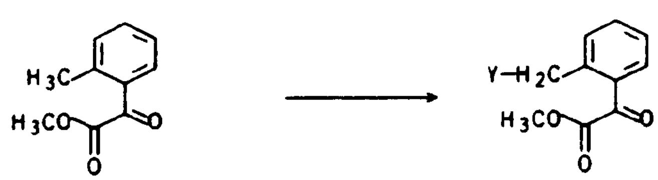

V VI: Y = Chlorid, Bromid

Substituierte Benzylverbindungen der allgemeinen Formel IIIb (X=CH, Y=Chlorid, Bromid) sind entweder bekannt oder können nach bekannten Verfahren hergestellt werden. Entsprechende Herstellverfahren sind z.B. beschrieben in DE-35 19 280, DE-35 45 318 und DE-35 45 319.

Substituierte Benzylverbindungen der allgemeinen Formel IIIc (X=CH oder N, Y=p-Toluolsulfonat, Methansulfonat, Trifluormethansulfonat) lassen sich aus den entsprechenden Verbindungen der allgemeinen Formel IIIa (X=N, Y=Chlorid, Bromid) oder IIIb (X=CH, Y=Chlorid, Bromid) durch Umsetzung mit p-Toluolsulfonsäure (Y=p-Toluolsulfonat), Methansulfonsäure (Y=Methansulfonat) oder Trifluormethansulfonsäure (Y=Trifluormethansulfonat) herstellen. Die Reaktionen können z.B. in einem inerten Lösungs- und Verdünnungsmittel (z.B. Dimethylformamid) in Gegenwart einer Base (z.B. Kaliumcarbonat) durchgeführt werden. Alternativ kann auch so vorgegangen werden, daß man die entsprechende Sulfonsäure zunächst in ihr Natrium-oder Kaliumsalz überführt und dieses dann in einem inerten Lösungs- und Verdünnungsmittel (z.B. Dimethylformamid) mit einer Verbindung der allgemeinen Formel IIIa oder IIIb zu den substituierten Benzylverbindungen der allgemeinen Formel IIIc umsetzt.

Die Darstellung der neuen Verbindungen der allgemeinen Formel I kann auch so erfolgen, daß man die neuen, substituierten Carbonylverbindungen der allgemeinen Formel VII mit einem substituierten Hydroxylamin der allgemeinen Formel VIII oder mit einem Säureadditionssalz (z.B. Hydrochlorid, Hydrobromid) von VIII umsetzt.

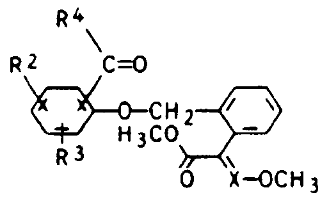

VII

+

$H_2N-OR^1$

VIII

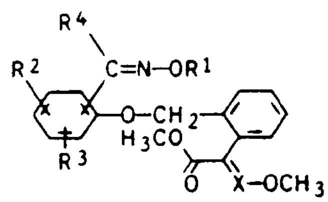

I

$R^1$, $R^2$, $R^3$, $R^4$ und X haben die oben genannten Bedeutungen.

Die Reaktion kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Natriumcarbonat, Kaliumcarbonat, Natriumhydrogencarbonat, Kaliumhydrogencarbonat, Natriumhydroxid, Kaliumhydroxid) zuzusetzen.

Als Ausgangsverbindungen benötigt man die neuen substituierten Carbonylverbindungen der allgemeinen Formel VII. Diese erhält man durch Umsetzung der substituierten Benzylverbindungen der allgemeinen Formel III, in der Y eine Abgangsgruppe (z.B. Chlorid, Bromid, p-Toluolsulfonat, Methansulfonat, Trifluormethansulfonat) bedeutet, mit substituierten Carbonylverbindungen der allgemeinen Formel IX. Die Verbindungen der allgemeinen Formel IX sind bekannt. Sie können nach bekannten und allgemein üblichen Verfahren und Methoden hergestellt werden.

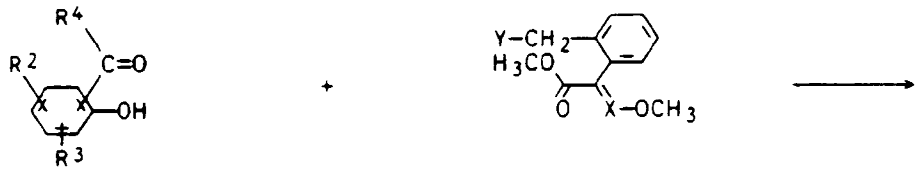

IX III

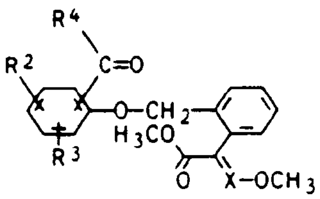

VII

$R^1$, $R^2$, $R^3$, $R^4$ und X haben die oben genannten Bedeutungen.

Die folgenden Beispiele und Vorschriften sollen die Herstellung der neuen Wirkstoffe und ihrer Vorprodukte erläutern.

Vorschrift 1

2-(Brommethyl)-phenylglyoxylsäuremethylester-O-methyloxim

27,5 g (0,133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 %) der oben genannten Verbindung als Öl erhalten.

Beispiel 1

2-(2'-Methoxyiminomethyl-phenoxymethyl)-phenylglyoxylsäure-methylester-O-methyloxim

3,0 g (20 mmol) 2-Hydroxy-benzaldehyd-O-methyloxim werden in 20 ml Methanol gelöst und mit 3,6 g (20 mmol) Natriummethanolat (30 %ig in Methanol) versetzt. Man kocht vier Stunden am Rückfluß und engt das Reaktionsgemisch ein. Der Rückstand wird in 100 ml Dimethylformamid aufgenommen und mit 6,5 g (23 mmol) 2-(Brommethyl)-phenylglyoxylsäuremethylester-O-methyloxim in 50 ml Dimethylformamid versetzt. Man rührt fünf Stunden bei 100°C, zieht das Lösungsmittel ab und nimmt den Rückstand in Essigsäureethylester auf. Die organische Phase wird mit Wasser gewaschen, getrocknet und eingeengt. Der Rückstand wird mit Pentan überschichtet und durch Anreiben kristallisiert. Man erhält 4,8 g (67 % der Theorie) der Titelverbindung in Form farbloser Kristalle (Fp.: 73-76°C, Verbindung Nr. 2).

Beispiel 2

α-[2-(2'-Ethoxyiminomethyl-phenoxymethyl)-phenyl]-β-methoxy-acrylsäure-methylester

6,9 g (42 mmol) 2-Hydroxy-benzaldehyd-O-ethyloxim und 10,0 g (35 mmol) α-(2-Brommethylphenyl)-β-methoxy-acrylsäuremethylester werden in 100 ml Dimethylformamid gelöst und mit 7,3 g (53 mmol) Kaliumcarbonat versetzt. Man rührt 48 Stunden bei Raumtemperatur, engt das Reaktionsgemisch ein und nimmt den Rückstand in Methylenchlorid auf. Die organische Phase wird mit Wasser gewaschen, über $MgSO_4$ getrocknet und eingeengt. Das erhaltene Öl wird durch Chromatographie über Kieselgel (Cyclohexan)/Essigester) gereinigt. Man erhält 8,4 g (65 % der Theorie) der Titelverbindung in Form farbloser Kristalle (Fp.: 86 - 88°C, Verbindung Nr. 23).

Beispiel 3

α-[2-(3'-(Ethoxyiminoeth-1"-yl)-phenoxymethyl)-phenyl]-β-methoxy-acrylsäuremethylester

7,5 g (0,042 mol) 3-(Ethoxyiminoeth-1'-yl)-phenol und 10,0 g (0,035 mol) α-(2-Brommethylphenyl)-β-methoxy-acrylsäuremethylester werden in 100 ml Dimethylformamid gelöst und mit 7,3 g (0,053 mol) Kaliumcarbonat versetzt. Man rührt 48 Stunden bei Raumtemperatur (20°C), hydrolysiert mit Wasser und extrahiert mit Diethylether. Die organische Phase wird mit Wasser gewaschen, über Magnesiumsulfat getrocknet und eingeengt. Das erhaltene Öl wird durch Chromatographie an Aluminiumoxid (Cyclohexan) gereinigt. Man erhält 7,3 g (54 %) der Titelverbindung als farbloses Öl (Verbindung Nr. 447).

Beispiel 4

2-[3'-(n-Butoxyiminoeth-1"-yl)phenoxymethyl]-phenylglyoxylsäure-methylester-O-methyloxim

a) 3,0 g (0,022 mol) 3-Hydroxy-acetophenon und 6,0 g (0,021 mol) 2-(Brommethyl)-phenylglyoxylsäuremethylester-O-methyloxim werden in 30 ml Dimethylformamid gelöst und mit 5,5 g (0,040 mol) Kaliumcarbonat versetzt. Man rührt 24 Stunden bei Raumtemperatur, hydrolysiert mit Wasser und extrahiert mit Methyl-tert.-butylether. Die organische Phase wird mit Wasser gewaschen, getrocknet und eingeengt. Man erhält 5,8 g (85 %) 2-(3'-Ethanoyl-phenoxymethyl)-phenylglyoxylsäuremethylester-O-methyloxim als farbloses Öl.
b) 5,8 g (0,017 mol) 2-(3'-Ethanoyl-phenoxymethyl)-phenylglyoxylsäure-methylester-O-methyloxim und 2,5 g (0,020 mol) n-Butoxyamin-hydrochlorid werden in 60 ml Methanol 24 Stunden bei Raumtemperatur gerührt. Anschließend hydrolysiert man mit Wasser und extrahiert mit Methyl-tert.-butylether. Die organische Phase wird mit Wasser gewaschen, getrocknet und eingeengt. Man erhält 5,0 g (71 %) der Titelverbindung als farbloses Öl (Verbindung 470).

Beispiel 5

2-[4'-(Benzyloxyiminoeth-1"-yl)-phenoxymethyl]-phenylglyoxylsäure-methylester-O-methyloxim

a) 3,0 g (0,022 mol) 4-Hydroxy-acetophenon und 6,0 g (0,021 mol) 2-(Brommethyl)-phenylglyoxylsäuremethylester-O-methyloxim werden in 30 ml Dimethylformamid gelöst und mit 5,5 g (0,040 mol) Kaliumcarbonat versetzt. Man rührt 24 Stunden bei Raumtemperatur, hydrolysiert mit Wasser und extrahiert mit Methyl-tert.-butylether. Die organische Phase wird mit Wasser gewaschen, getrocknet und eingeengt. Man erhält 5,3 g (78 %) 2-(4'-Ethanoyl-phenoxymethyl)-phenylglyoxylsäuremethylester-O-methyloxim als farbloses Öl.
b) 5,3 g (0,016 mol) 2-(4'-Ethanoyl-phenoxymethyl)-phenylglyoxylsäure-methylester-O-methyloxim und 3,0 g (0,019 mol) Benzyloxyamin-hydrochlorid werden in 60 ml Methanol 24 Stunden bei Raumtemperatur gerührt. Anschließend hydrolysiert man mit Wasser und extrahiert mit Methyl-tert.-butylether. Die organische Phase wird mit Wasser gewaschen, getrocknet und eingeengt. Man erhält 5,9 g (83 %) der Titelverbindung in Form farbloser Kristalle (Fp.: 104-106°C, Verbindung Nr. 590).
In entsprechender Weise lassen sich die in den Tabellen 1 bis 3 aufgeführten Verbindungen herstellen.

Tabelle 1

Verbindungen der allgemeinen Formel Ia (2-Stellung des Oximethers). Die Konfigurationsangabe (E) bezieht sich auf die β-Methoxy-acrylsäuremethylestergruppe bzw. auf die Glyoxylsäuremethylester-O-methyloximgruppe.

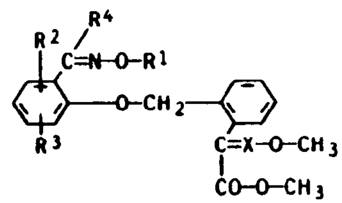

Ia

| Verb.-Nr. | $R^1$ | $R^2$ | $R^3$ | $R^4$ | X | Fp.(°C) |
|---|---|---|---|---|---|---|
| 1 | $CH_3$- | H | H | H | CH | 82-84 (E) |
| 2 | $CH_3$- | H | H | H | N | 73-76 (E) |
| 3 | $CH_3$- | 4-Cl | H | H | CH | |
| 4 | $CH_3$- | 4-Cl | H | H | N | |
| 5 | $CH_3$- | 4-$CH_3$ | H | H | CH | |
| 6 | $CH_3$- | 4-$CH_3$ | H | H | N | |
| 7 | $CH_3$- | 4-$OCH_3$ | H | H | CH | |
| 8 | $CH_3$- | 4-$OCH_3$ | H | H | N | |
| 9 | $CH_3$- | 5-F | H | H | CH | |
| 10 | $CH_3$- | 5-F | H | H | N | |
| 11 | $CH_3$- | 5-Cl | H | H | CH | |
| 12 | $CH_3$- | 5-Cl | H | H | N | |
| 13 | $CH_3$- | 5-Br | H | H | CH | |
| 14 | $CH_3$- | 5-Br | H | H | N | |
| 15 | $CH_3$- | 5-$CH_3$ | H | H | CH | |
| 16 | $CH_3$- | 5-$CH_3$ | H | H | N | |

| Verb.-Nr. | $R^1$ | $R^2$ | $R^3$ | $R^4$ | X | Fp. (°C) |
|---|---|---|---|---|---|---|
| 17 | $CH_3$- | 5-$OCH_3$ | H | H | CH | |
| 18 | $CH_3$- | 5-$OCH_3$ | H | H | N | |
| 19 | $CH_3$- | 6-$OCH_3$ | H | H | CH | |
| 20 | $CH_3$- | 6-$OCH_3$ | H | H | N | |
| 21 | $CH_3$- | 4-Cl | 6-Cl | H | CH | |
| 22 | $CH_3$- | 4-Cl | 6-Cl | H | N | |
| 23 | $CH_3$-$CH_2$- | H | H | H | CH | 86-88 (E) |
| 24 | $CH_3$-$CH_2$- | H | H | H | N | 89-90 (E) |
| 25 | $CH_3$-$CH_2$- | 4-Cl | H | H | CH | 95-97 (E) |
| 26 | $CH_3$-$CH_2$- | 4-Cl | H | H | N | |
| 27 | $CH_3$-$CH_2$- | 4-$CH_3$ | H | H | CH | |
| 28 | $CH_3$-$CH_2$- | 4-$CH_3$ | H | H | N | |
| 29 | $CH_3$-$CH_2$- | 4-$OCH_3$ | H | H | CH | |
| 30 | $CH_3$-$CH_2$- | 4-$OCH_3$ | H | H | N | |
| 31 | $CH_3$-$CH_2$- | 5-F | H | H | CH | |
| 32 | $CH_3$-$CH_2$- | 5-F | H | H | N | |
| 33 | $CH_3$-$CH_2$- | 5-Cl | H | H | CH | |
| 34 | $CH_3$-$CH_2$- | 5-Cl | H | H | N | |
| 35 | $CH_3$-$CH_2$- | 5-Br | H | H | CH | |
| 36 | $CH_3$-$CH_2$- | 5-Br | H | H | N | |
| 37 | $CH_3$-$CH_2$- | 5-$CH_3$ | H | H | CH | |
| 38 | $CH_3$-$CH_2$- | 5-$CH_3$ | H | H | N | |
| 39 | $CH_3$-$CH_2$- | 5-$OCH_3$ | H | H | CH | |
| 40 | $CH_3$-$CH_2$- | 5-$OCH_3$ | H | H | N | |
| 41 | $CH_3$-$CH_2$- | 6-$OCH_3$ | H | H | CH | |
| 42 | $CH_3$-$CH_2$- | 6-$OCH_3$ | H | H | N | |
| 43 | $CH_3$-$CH_2$- | 4-Cl | 6-Cl | H | CH | |

| Verb.-Nr. | $R^1$ | $R^2$ | $R^3$ | $R^4$ | X | Fp. (°C) |
|---|---|---|---|---|---|---|
| 44 | $CH_3-CH_2-$ | 4-Cl | 6-Cl | H | N | |
| 45 | $CH_3-CH_2-CH_2-$ | H | H | H | CH | |
| 46 | $CH_3-CH_2-CH_2-$ | H | H | H | N | |
| 47 | $CH_2=CH-CH_2-$ | H | H | H | CH | |
| 48 | $CH_2=CH-CH_2-$ | H | H | H | N | |
| 49 | $CH_3-CH(CH_3)-$ | H | H | H | CH | |
| 50 | $CH_3-CH(CH_3)-$ | H | H | H | N | |
| 51 | $HC\equiv C-CH_2-$ | H | H | H | CH | |
| 52 | $HC\equiv C-CH_2-$ | H | H | H | N | |
| 53 | cyclo-$C_3H_5-CH_2-$ | H | H | H | CH | |
| 54 | cyclo-$C_3H_5-CH_2-$ | H | H | H | N | |
| 55 | $CH_3-CH_2-CH_2-CH_2-$ | H | H | H | CH | |
| 56 | $CH_3-CH_2-CH_2-CH_2-$ | H | H | H | N | |
| 57 | $CH_3-CH=CH-CH_2-$ | H | H | H | CH | |
| 58 | $CH_3-CH=CH-CH_2-$ | H | H | H | N | |
| 59 | $CH_3-(CH_2)_5-$ | H | H | H | CH | |
| 60 | $CH_3-(CH_2)_5-$ | H | H | H | N | |
| 61 | cyclo-$C_6H_{11}$ | H | H | H | CH | |
| 62 | cyclo-$C_6H_{11}-$ | H | H | H | N | |
| 63 | $C_6H_5-CH_2-$ | H | H | H | CH | |
| 64 | $C_6H_5-CH_2-$ | H | H | H | N | |
| 65 | $4-Cl-C_6H_4-CH_2-$ | H | H | H | CH | |
| 66 | $4-Cl-C_6H_4-CH_2-$ | H | H | H | N | |

| Verb.-Nr. | $R^1$ | $R^2$ | $R^3$ | $R^4$ | X | Fp.(°C) |
|---|---|---|---|---|---|---|
| 67 | $3-CF_3-C_6H_4-CH_2-$ | H | H | H | CH | |
| 68 | $3-CF_3-C_6H_4-CH_2-$ | H | H | H | N | |
| 69 | $4-Cl-C_6H_4-CH_2-CH_2-$ | H | H | H | CH | |
| 70 | $4-Cl-C_6H_4-CH_2-CH_2-$ | H | H | H | N | |
| 71 | $C_6H_5-CH_2-CH_2-CH_2-$ | H | H | H | CH | |
| 72 | $C_6H_5-CH_2-CH_2-CH_2-$ | H | H | H | N | |
| 73 | $C_6H_5-(CH_2)_4-$ | H | H | H | CH | |
| 74 | $C_6H_5-(CH_2)_4-$ | H | H | H | N | |
| 75 | $C_6H_5-CH_2-CH=CH-CH_2-$ | H | H | H | CH | |
| 76 | $C_6H_5-CH_2-CH=CH-CH_2-$ | H | H | H | N | |
| 77 | $4-F-C_6H_4-CH=CH-CH_2-CH_2-$ | H | H | H | CH | |
| 78 | $4-F-C_6H_4-CH=CH-CH_2-CH_2-$ | H | H | H | N | |
| 79 | $t-C_4H_9O-CO-CH_2-$ | H | H | H | CH | |
| 80 | $t-C_4H_9O-CO-CH_2-$ | H | H | H | N | |
| 81 | $t-C_4H_9O-CO-(CH_2)_3-$ | H | H | H | CH | |
| 82 | $t-C_4H_9O-CO-(CH_2)_3-$ | H | H | H | N | |
| 83 | $Cl-CH=CH-CH_2-$ | H | H | H | CH | |
| 84 | $Cl-CH=CH-CH_2-$ | H | H | H | N | |
| 221 | $C_2H_5$ | $6-OC_2H_5$ | H | H | CH | |
| 222 | $C_2H_5$ | $6-OC_2H_5$ | H | H | N | |
| 223 | $CH_3-C(CH_3)_2-$ | H | H | H | CH | |
| 224 | $CH_3-C(CH_3)_2-$ | H | H | H | N | |
| 225 | $CH_3-CH(CH_3)-CH_2-$ | H | H | H | CH | |
| 226 | $CH_3-CH(CH_3)-CH_2-$ | H | H | H | N | |
| 227 | $CH_2=C(CH_3)-CH_2$ | H | H | H | CH | |

| Verb.-Nr. | $R^1$ | $R^2$ | $R^3$ | $R^4$ | X | Fp.(°C) |
|---|---|---|---|---|---|---|
| 228 | $CH_2=C(CH_3)-CH_2$ | H | H | H | N | |
| 229 | $CH_3-CH(CH_3)-CH_2-CH_2-$ | H | H | H | CH | |
| 230 | $CH_3-CH(CH_3)-CH_2-CH_2-$ | H | H | H | N | |
| 231 | $CH_3-(CH_2)_4-$ | H | H | H | CH | |
| 232 | $CH_3-(CH_2)_4-$ | H | H | H | N | |
| 233 | $2-F-C_6H_4-CH_2-$ | H | H | H | CH | |
| 234 | $2-F-C_6H_4-CH_2-$ | H | H | H | N | |
| 235 | $3-F-C_6H_4-CH_2-$ | H | H | H | CH | |
| 236 | $3-F-C_6H_4-CH_2-$ | H | H | H | N | |
| 237 | $2-Cl-C_6H_4-CH_2-$ | H | H | H | CH | |
| 238 | $2-Cl-C_6H_4-CH_2-$ | H | H | H | N | |
| 239 | $3,4-Cl_2-C_6H_3-CH_2-$ | H | H | H | CH | |
| 240 | $3,4-Cl_2-C_6H_3-CH_2-$ | H | H | H | N | |
| 241 | $2,6-Cl_2-C_6H_3-CH_2-$ | H | H | H | CH | |
| 242 | $2,6-Cl_2-C_6H_3-CH_2-$ | H | H | H | N | |
| 243 | $C_6H_5-CH_2-CH_2-$ | H | H | H | CH | |
| 244 | $C_6H_5-CH_2-CH_2-$ | H | H | H | N | |
| 245 | $C_6H_5-CH=CH-CH_2-CH_2-$ | H | H | H | CH | |
| 246 | $C_6H_5-CH=CH-CH_2-CH_2-$ | H | H | H | N | |
| 247 | $4-Cl-C_6H_4-CH_2-CH=CH-CH_2-$ | H | H | H | CH | |
| 248 | $4-Cl-C_6H_4-CH_2-CH=CH-CH_2-$ | H | H | H | N | |
| 249 | $4-CF_3-C_6H_4-CH_2-CH=CH-CH_2-$ | H | H | H | CH | |
| 250 | $4-CF_3-C_6H_4-CH_2-CH=CH-CH_2-$ | H | H | H | N | |

| Verb.-Nr. | $R^1$ | $R^2$ | $R^3$ | $R^4$ | X | Fp. (°C) |
|---|---|---|---|---|---|---|
| 311 | $CH_3$ | H | H | $CH_3$ | CH | |
| 312 | $CH_3$ | H | H | $CH_3$ | N | |
| 313 | $CH_3$ | 4-Cl | H | $CH_3$ | CH | |
| 314 | $CH_3$ | 4-Cl | H | $CH_3$ | N | |
| 315 | $CH_3$ | 4-$CH_3$ | H | $CH_3$ | CH | |
| 316 | $CH_3$ | 4-$CH_3$ | H | $CH_3$ | N | |
| 317 | $CH_3$ | 4-$OCH_3$ | H | $CH_3$ | CH | |
| 318 | $CH_3$ | 4-$OCH_3$ | H | $CH_3$ | N | |
| 319 | $CH_3$ | 5-F | H | $CH_3$ | CH | |
| 320 | $CH_3$ | 5-F | H | $CH_3$ | N | |
| 321 | $CH_3$ | 5-Cl | H | $CH_3$ | CH | |
| 322 | $CH_3$ | 5-Cl | H | $CH_3$ | N | |
| 323 | $CH_3$ | 5-Br | H | $CH_3$ | CH | |
| 324 | $CH_3$ | 5-Br | H | $CH_3$ | N | |
| 325 | $CH_3$ | 5-$CH_3$ | H | $CH_3$ | CH | |
| 326 | $CH_3$ | 5-$CH_3$ | H | $CH_3$ | N | |
| 327 | $CH_3$ | 5-$OCH_3$ | H | $CH_3$ | CH | |
| 328 | $CH_3$ | 5-$OCH_3$ | H | $CH_3$ | N | |
| 329 | $CH_3$ | 6-$OCH_3$ | H | $CH_3$ | CH | |

| Verb.-Nr. | $R^1$ | $R^2$ | $R^3$ | $R^4$ | X | Fp. (°C) |
|---|---|---|---|---|---|---|
| 330 | $CH_3$ | 6-$OCH_3$ | H | $CH_3$ | N | |
| 331 | $CH_3$ | 4-Cl | 6-Cl | $CH_3$ | CH | |
| 332 | $CH_3$ | 4-Cl | 6-Cl | $CH_3$ | N | |
| 333 | $CH_3$-$CH_2$ | H | H | $CH_3$ | CH | |
| 334 | $CH_3$-$CH_2$ | H | H | $CH_3$ | N | |
| 335 | $CH_3$-$CH_2$ | 4-Cl | H | $CH_3$ | CH | |
| 336 | $CH_3$-$CH_2$ | 4-Cl | H | $CH_3$ | N | |
| 337 | $CH_3$-$CH_2$ | 4-$CH_3$ | H | $CH_3$ | CH | |
| 338 | $CH_3$-$CH_2$ | 4-$CH_3$ | H | $CH_3$ | N | |
| 339 | $CH_3$-$CH_2$ | 4-$OCH_3$ | H | $CH_3$ | CH | |
| 340 | $CH_3$-$CH_2$ | 4-$OCH_3$ | H | $CH_3$ | N | |
| 341 | $CH_3$-$CH_2$ | 5-F | H | $CH_3$ | CH | |
| 342 | $CH_3$-$CH_2$ | 5-F | H | $CH_3$ | N | |
| 343 | $CH_3$-$CH_2$ | 5-Cl | H | $CH_3$ | CH | |
| 344 | $CH_3$-$CH_2$ | 5-Cl | H | $CH_3$ | N | |
| 345 | $CH_3$-$CH_2$ | 5-Br | H | $CH_3$ | CH | |
| 346 | $CH_3$-$CH_2$ | 5-Br | H | $CH_3$ | N | |
| 347 | $CH_3$-$CH_2$ | 5-$CH_3$ | H | $CH_3$ | CH | |

| Verb.-Nr. | $R^1$ | $R^2$ | $R^3$ | $R^4$ | X | Fp. (°C) |
|---|---|---|---|---|---|---|
| 348 | $CH_3-CH_2$ | $5-CH_3$ | H | $CH_3$ | N | |
| 349 | $CH_3-CH_2$ | $5-OCH_3$ | H | $CH_3$ | CH | |
| 350 | $CH_3-CH_2$ | $5-OCH_3$ | H | $CH_3$ | N | |
| 351 | $CH_3-CH_2$ | $6-OCH_3$ | H | $CH_3$ | CH | |
| 352 | $CH_3-CH_2$ | $6-OCH_3$ | H | $CH_3$ | N | |
| 353 | $CH_3-CH_2$ | 4-Cl | 6-Cl | $CH_3$ | CH | |
| 354 | $CH_3-CH_2$ | 4-Cl | 6-Cl | $CH_3$ | N | |
| 355 | $CH_3-CH_2-CH_2$ | H | H | $CH_3$ | CH | |
| 356 | $CH_3-CH_2-CH_2$ | H | H | $CH_3$ | N | |
| 357 | $CH_2=CH-CH_2$ | H | H | $CH_3$ | CH | |
| 358 | $CH_2=CH-CH_2$ | H | H | $CH_3$ | N | |
| 359 | $CH_3-CH(CH_3)$ | H | H | $CH_3$ | CH | |
| 360 | $CH_3-CH(CH_3)$ | H | H | $CH_3$ | N | |
| 361 | $HC{\equiv}C-CH_2$ | H | H | $CH_3$ | CH | |
| 362 | $HC{\equiv}C-CH_2$ | H | H | $CH_3$ | N | |
| 363 | cyclo-$C_3H_5-CH_2$ | H | H | $CH_3$ | CH | |
| 364 | cyclo-$C_3H_5-CH_2$ | H | H | $CH_3$ | N | |
| 365 | $CH_3-CH_2-CH_2-CH_2$ | H | H | $CH_3$ | CH | |
| 366 | $CH_3-CH_2-CH_2-CH_2$ | H | H | $CH_3$ | N | |
| 367 | $CH_3-CH=CH-CH_2$ | H | H | $CH_3$ | CH | |

| Verb.-Nr. | $R^1$ | $R^2$ | $R^3$ | $R^4$ | X | Fp. (°C) |
|---|---|---|---|---|---|---|
| 368 | $CH_3-CH=CH-CH_2$ | H | H | $CH_3$ | N | |
| 369 | $CH_3-(CH_2)_5$ | H | H | $CH_3$ | CH | |
| 370 | $CH_3-(CH_2)_5$ | H | H | $CH_3$ | N | |
| 371 | cyclo-$C_6H_{11}$ | H | H | $CH_3$ | CH | |
| 372 | cyclo-$C_6H_{11}$ | H | H | $CH_3$ | N | |
| 373 | $C_6H_5-CH_2$ | H | H | $CH_3$ | CH | |
| 374 | $C_6H_5-CH_2$ | H | H | $CH_3$ | N | |
| 375 | $4-Cl-C_6H_4-CH_2$ | H | H | $CH_3$ | CH | |
| 376 | $4-Cl-C_6H_4-CH_2$ | H | H | $CH_3$ | N | |
| 377 | $3-CF_3-C_6H_4-CH_2$ | H | H | $CH_3$ | CH | |
| 378 | $3-CF_3-C_6H_4-CH_2$ | H | H | $CH_3$ | N | |
| 379 | $4-Cl-C_6H_4-CH_2-CH_2$ | H | H | $CH_3$ | CH | |
| 380 | $4-Cl-C_6H_4-CH_2-CH_2$ | H | H | $CH_3$ | N | |
| 381 | $C_6H_5-CH_2-CH_2-CH_2$ | H | H | $CH_3$ | CH | |
| 382 | $C_6H_5-CH_2-CH_2-CH_2$ | H | H | $CH_3$ | N | |
| 383 | $C_6H_5-(CH_2)_4$ | H | H | $CH_3$ | CH | |
| 384 | $C_6H_5-(CH_2)_4$ | H | H | $CH_3$ | N | |
| 385 | $C_6H_5-CH_2-CH=CH-CH_2$ | H | H | $CH_3$ | CH | |
| 386 | $C_6H_5-CH_2-CH=CH-CH_2$ | H | H | $CH_3$ | N | |
| 387 | $4-F-C_6H_4-CH=CH-CH_2-CH_2$ | H | H | $CH_3$ | CH | |
| 388 | $4-F-C_6H_4-CH=CH-CH_2-CH_2$ | H | H | $CH_3$ | N | |
| 389 | $t-C_4H_9O-CO-CH_2$ | H | H | $CH_3$ | CH | |

| Verb.-Nr. | $R^1$ | $R^2$ | $R^3$ | $R^4$ | X | Fp. (°C) |
|---|---|---|---|---|---|---|
| 390 | $t-C_4H_9O-CO-CH_2$ | H | H | $CH_3$ | N | |
| 391 | $t-C_4H_9O-CO-(CH_2)_3$ | H | H | $CH_3$ | CH | |
| 392 | $t-C_4H_9O-CO-(CH_2)_3$ | H | H | $CH_3$ | N | |
| 393 | $Cl-CH=CH-CH_2$ | H | H | $CH_3$ | CH | |
| 394 | $Cl-CH=CH-CH_2$ | H | H | $CH_3$ | N | |
| 395 | $C_2H_5$ | $6-OC_2H_5$ | H | $CH_3$ | CH | |
| 396 | $C_2H_5$ | $6-OC_2H_5$ | H | $CH_3$ | N | |
| 397 | $CH_3-C(CH_3)_2$ | H | H | $CH_3$ | CH | |
| 398 | $CH_3-C(CH_3)_2$ | H | H | $CH_3$ | N | |
| 399 | $CH_3-CH(CH_3)-CH_2$ | H | H | $CH_3$ | CH | |
| 400 | $CH_3-CH(CH_3)-CH_2$ | H | H | $CH_3$ | N | |
| 401 | $CH_2=C(CH_3)-CH_2$ | H | H | $CH_3$ | CH | |
| 402 | $CH_2=C(CH_3)-CH_2$ | H | H | $CH_3$ | N | |
| 403 | $CH_3-CH(CH_3)-CH_2-CH_2$ | H | H | $CH_3$ | CH | |
| 404 | $CH_3-CH(CH_3)-CH_2-CH_2$ | H | H | $CH_3$ | N | |
| 405 | $CH_3-(CH_2)_4$ | H | H | $CH_3$ | CH | |
| 406 | $CH_3-(CH_2)_4$ | H | H | $CH_3$ | N | |
| 407 | $2-F-C_6H_4-CH_2$ | H | H | $CH_3$ | CH | |
| 408 | $2-F-C_6H_4-CH_2$ | H | H | $CH_3$ | N | |
| 409 | $3-F-C_6H_4-CH_2$ | H | H | $CH_3$ | CH | |
| 410 | $3-F-C_6H_4-CH_2$ | H | H | $CH_3$ | N | |
| 411 | $2-Cl-C_6H_4-CH_2$ | H | H | $CH_3$ | CH | |

| Verb.-Nr. | $R^1$ | $R^2$ | $R^3$ | $R^4$ | X | Fp. (°C) |
|---|---|---|---|---|---|---|
| 412 | $2-Cl-C_6H_4-CH_2$ | H | H | $CH_3$ | N | |
| 413 | $3,4-Cl_2-C_6H_3-CH_2$ | H | H | $CH_3$ | CH | |
| 414 | $3,4-Cl_2-C_6H_3-CH_2$ | H | H | $CH_3$ | N | |
| 415 | $2,6-Cl_2-C_6H_3-CH_2$ | H | H | $CH_3$ | CH | |
| 416 | $2,6-Cl_2-C_6H_3-CH_2$ | H | H | $CH_3$ | N | |
| 417 | $C_6H_5-CH_2-CH_2$ | H | H | $CH_3$ | CH | |
| 418 | $C_6H_5-CH_2-CH_2$ | H | H | $CH_3$ | N | |
| 419 | $C_6H_5-CH=CH-CH_2-CH_2$ | H | H | $CH_3$ | CH | |
| 420 | $C_6H_5-CH=CH-CH_2-CH_2$ | H | H | $CH_3$ | N | |
| 421 | $4-Cl-C_6H_4-CH_2-CH=CH-CH_2$ | H | H | $CH_3$ | CH | |
| 422 | $4-Cl-C_6H_4-CH_2-CH=CH-CH_2$ | H | H | $CH_3$ | N | |
| 423 | $4-CF_3-C_6H_4-CH_2-CH=CH-CH_2$ | H | H | $CH_3$ | CH | |
| 424 | $4-CF_3-C_6H_4-CH_2-CH=CH-CH_2$ | H | H | $CH_3$ | N | |
| 425 | $CH_3$ | H | H | $C_6H_5$ | CH | |
| 426 | $CH_3$ | H | H | $C_6H_5$ | N | |
| 427 | $C_2H_5$ | H | H | $C_6H_5$ | CH | |
| 428 | $C_2H_5$ | H | H | $C_6H_5$ | N | |
| 429 | $CH_3-CH_2-CH_2$ | H | H | $C_6H_5$ | CH | |
| 430 | $CH_3-CH_2-CH_2$ | H | H | $C_6H_5$ | N | |
| 431 | $CH_3-(CH_2)_5$ | H | H | $C_6H_5$ | CH | |
| 432 | $CH_3-(CH_2)_5$ | H | H | $C_6H_5$ | N | |
| 433 | $C_6H_5-CH_2$ | H | H | $C_6H_5$ | CH | |
| 434 | $C_6H_5-CH_2$ | H | H | $C_6H_5$ | N | |

Tabelle 2

Verbindungen der allgemeinen Formel Ib (3-Stellung des Oximethers). Die Konfigurationsangabe bezieht sich auf die β-Methoxy-acrylsäuremethylestergruppe bzw. auf die Glyoxylsäuremethylester-O-methyloximgruppe.

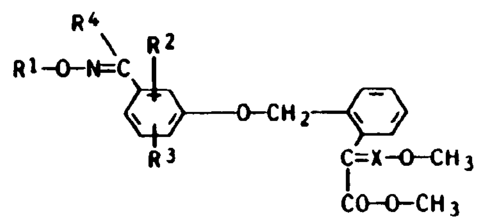

Ib

| Verb.-Nr. | $R^1$ | $R^2$ | $R^3$ | $R^4$ | X | Fp. (°C) |
|---|---|---|---|---|---|---|
| 85 | $CH_3$- | H | H | H | CH | 75-77 (E) |
| 86 | $CH_3$- | H | H | H | N | Öl (E) |
| 87 | $CH_3$- | 2-Cl | 5-Cl | H | CH | |
| 88 | $CH_3$- | 2-Cl | 5-Cl | H | N | |
| 89 | $CH_3$- | 4-Cl | H | H | CH | |
| 90 | $CH_3$- | 4-Cl | H | H | N | |
| 91 | $CH_3$- | 4-$CH_3$ | H | H | CH | |
| 92 | $CH_3$- | 4-$CH_3$ | H | H | N | |
| 93 | $CH_3$- | 5-$OCH_3$ | H | H | CH | |
| 94 | $CH_3$- | 5-$OCH_3$ | H | H | N | |
| 95 | $CH_3$- | 6-$OCH_3$ | H | H | CH | |
| 96 | $CH_3$- | 6-$OCH_3$ | H | H | N | |
| 97 | $CH_3$-$CH_2$- | H | H | H | CH | Öl (E) |
| 98 | $CH_3$-$CH_2$- | H | H | H | N | Öl (E) |

| Verb.-Nr. | $R^1$ | $R^2$ | $R^3$ | $R^4$ | X | Fp.(°C) |
|---|---|---|---|---|---|---|
| 99 | $CH_3-CH_2-$ | 2-Cl | 5-Cl | H | CH | |
| 100 | $CH_3-CH_2-$ | 2-Cl | 5-Cl | H | N | |
| 101 | $CH_3-CH_2-$ | 4-Cl | H | H | CH | |
| 102 | $CH_3-CH_2-$ | 4-Cl | H | H | N | |
| 103 | $CH_3-CH_2-$ | 4-$CH_3$ | H | H | CH | |
| 104 | $CH_3-CH_2-$ | 4-$CH_3$ | H | H | N | |
| 105 | $CH_3-CH_2-$ | 5-$OCH_3$ | H | H | CH | |
| 106 | $CH_3-CH_2-$ | 5-$OCH_3$ | H | H | N | |
| 107 | $CH_3-CH_2-$ | 6-$OCH_3$ | H | H | CH | 96- 98 (E) |
| 108 | $CH_3-CH_2-$ | 6-$OCH_3$ | H | H | N | 124-126 (E) |
| 109 | $CH_3-CH_2-CH_2-$ | H | H | H | CH | |
| 110 | $CH_3-CH_2-CH_2-$ | H | H | H | N | |
| 111 | $CH_2=CH-CH_2-$ | H | H | H | CH | Öl (E) |
| 112 | $CH_2=CH-CH_2-$ | H | H | H | N | Öl (E) |
| 113 | $CH_3-CH(CH_3)-$ | H | H | H | CH | Öl (E) |
| 114 | $CH_3-CH(CH_3)-$ | H | H | H | N | Öl (E) |
| 115 | $HC{\equiv}C-CH_2-$ | H | H | H | CH | |
| 116 | $HC{\equiv}C-CH_2-$ | H | H | H | N | |
| 117 | cyclo-$C_3H_5-CH_2-$ | H | H | H | CH | |
| 118 | cyclo-$C_3H_5-CH_2-$ | H | H | H | N | |
| 119 | $CH_3-CH_2-CH_2-CH_2-$ | H | H | H | CH | Öl (E) |
| 120 | $CH_3-CH_2-CH_2-CH_2-$ | H | H | H | N | Öl (E) |
| 121 | $CH_3-CH=CH-CH_2-$ | H | H | H | CH | |

| Verb.-Nr. | $R^1$ | $R^2$ | $R^3$ | $R^4$ | X | Fp. (°C) |
|---|---|---|---|---|---|---|
| 122 | $CH_3-CH=CH-CH_2-$ | H | H | H | N | |
| 123 | $CH_3-(CH_2)_5-$ | H | H | H | CH | Öl (E) |
| 124 | $CH_3-(CH_2)_5-$ | H | H | H | N | Öl (E) |
| 125 | cyclo-$C_6H_{11}$ | H | H | H | CH | |
| 126 | cyclo-$C_6H_{11}-$ | H | H | H | N | |
| 127 | $C_6H_5-CH_2-$ | H | H | H | CH | Öl (E) |
| 128 | $C_6H_5-CH_2-$ | H | H | H | N | Öl (E) |
| 129 | $4-Cl-C_6H_4-CH_2-$ | H | H | H | CH | |
| 130 | $4-Cl-C_6H_4-CH_2-$ | H | H | H | N | |
| 131 | $3-CF_3-C_6H_4-CH_2-$ | H | H | H | CH | |
| 132 | $3-CF_3-C_6H_4-CH_2-$ | H | H | H | N | |
| 133 | $4-Cl-C_6H_4-CH_2-CH_2-$ | H | H | H | CH | |
| 134 | $4-Cl-C_6H_4-CH_2-CH_2-$ | H | H | H | N | |
| 135 | $C_6H_5-CH_2-CH_2-CH_2-$ | H | H | H | CH | |
| 136 | $C_6H_5-CH_2-CH_2-CH_2-$ | H | H | H | N | |
| 137 | $C_6H_5-(CH_2)_4-$ | H | H | H | CH | |
| 138 | $C_6H_5-(CH_2)_4-$ | H | H | H | N | |
| 139 | $C_6H_5-CH_2-CH=CH-CH_2-$ | H | H | H | CH | |
| 140 | $C_6H_5-CH_2-CH=CH-CH_2-$ | H | H | H | N | |
| 141 | $4-F-C_6H_4-CH=CH-CH_2-CH_2-$ | H | H | H | CH | |
| 142 | $4-F-C_6H_4-CH=CH-CH_2-CH_2-$ | H | H | H | N | |
| 143 | $t-C_4H_9O-CO-CH_2-$ | H | H | H | CH | |
| 144 | $t-C_4H_9O-CO-CH_2-$ | H | H | H | N | |

| Verb.-Nr. | $R^1$ | $R^2$ | $R^3$ | $R^4$ | X | Fp.(°C) |
|---|---|---|---|---|---|---|
| 145 | $t-C_4H_9O-CO-(CH_2)_3-$ | H | H | H | CH | |
| 146 | $t-C_4H_9O-CO-(CH_2)_3-$ | H | H | H | N | |
| 147 | $Cl-CH=CH-CH_2-$ | H | H | H | CH | |
| 148 | $Cl-CH=CH-CH_2-$ | H | H | H | N | |
| 251 | $C_2H_5$ | $6-OC_2H_5$ | H | H | CH | 83- 85 (E) |
| 252 | $C_2H_5$ | $6-OC_2H_5$ | H | H | N | 104-106 (E) |
| 253 | $CH_3-C(CH_3)_2-$ | H | H | H | CH | |
| 254 | $CH_3-C(CH_3)_2-$ | H | H | H | N | |
| 255 | $CH_3-CH(CH_3)-CH_2-$ | H | H | H | CH | |
| 256 | $CH_3-CH(CH_3)-CH_2-$ | H | H | H | N | |
| 257 | $CH_2=C(CH_3)-CH_2$ | H | H | H | CH | |
| 258 | $CH_2=C(CH_3)-CH_2$ | H | H | H | N | |
| 259 | $CH_3-CH(CH_3)-CH_2-CH_2-$ | H | H | H | CH | |
| 260 | $CH_3-CH(CH_3)-CH_2-CH_2-$ | H | H | H | N | |
| 261 | $CH_3-(CH_2)_4-$ | H | H | H | CH | |
| 262 | $CH_3-(CH_2)_4-$ | H | H | H | N | Öl (E) |
| 263 | $2-F-C_6H_4-CH_2-$ | H | H | H | CH | Öl (E) |
| 264 | $2-F-C_6H_4-CH_2-$ | H | H | H | N | Öl (E) |
| 265 | $3-F-C_6H_4-CH_2-$ | H | H | H | CH | Öl (E) |
| 266 | $3-F-C_6H_4-CH_2-$ | H | H | H | N | Öl (E) |
| 267 | $2-Cl-C_6H_4-CH_2-$ | H | H | H | CH | Öl (E) |
| 268 | $2-Cl-C_6H_4-CH_2-$ | H | H | H | N | |
| 269 | $3,4-Cl_2-C_6H_3-CH_2-$ | H | H | H | CH | Öl (E) |

| Verb.-Nr. | $R^1$ | $R^2$ | $R^3$ | $R^4$ | X | Fp. (°C) |
|---|---|---|---|---|---|---|
| 270 | $3,4-Cl_2-C_6H_3-CH_2-$ | H | H | H | N | Öl (E) |
| 271 | $2,6-Cl_2-C_6H_3-CH_2-$ | H | H | H | CH | Öl (E) |
| 272 | $2,6-Cl_2-C_6H_3-CH_2-$ | H | H | H | N | Öl (E) |
| 273 | $C_6H_5-CH_2-CH_2-$ | H | H | H | CH | Öl (E) |
| 274 | $C_6H_5-CH_2-CH_2-$ | H | H | H | N | Öl (E) |
| 275 | $C_6H_5-CH=CH-CH_2-CH_2-$ | H | H | H | CH | Öl (E) |
| 276 | $C_6H_5-CH=CH-CH_2-CH_2-$ | H | H | H | N | Öl (E) |
| 277 | $4-Cl-C_6H_4-CH_2-CH=CH-CH_2-$ | H | H | H | CH | Öl (E) |
| 278 | $4-Cl-C_6H_4-CH_2-CH=CH-CH_2-$ | H | H | H | N | Öl (E) |
| 279 | $4-CF_3-C_6H_4-CH_2-CH=CH-CH_2-$ | H | H | H | CH | Öl (E) |
| 280 | $4-CF_3-C_6H_4-CH_2-CH=CH-CH_2-$ | H | H | H | N | Öl (E) |
| 435 | $CH_3$ | H | H | $CH_3$ | CH | Öl (E) |
| 436 | $CH_3$ | H | H | $CH_3$ | N | Öl (E) |
| 437 | $CH_3$ | 2-Cl | 5-Cl | $CH_3$ | CH | |
| 438 | $CH_3$ | 2-Cl | 5-Cl | $CH_3$ | N | |
| 439 | $CH_3$ | 4-Cl | H | $CH_3$ | CH | |
| 440 | $CH_3$ | 4-Cl | H | $CH_3$ | N | |
| 441 | $CH_3$ | $4-CH_3$ | H | $CH_3$ | CH | |
| 442 | $CH_3$ | $4-CH_3$ | H | $CH_3$ | N | |
| 443 | $CH_3$ | $5-OCH_3$ | H | $CH_3$ | CH | |
| 444 | $CH_3$ | $5-OCH_3$ | H | $CH_3$ | N | |
| 445 | $CH_3$ | $6-OCH_3$ | H | $CH_3$ | CH | |
| 446 | $CH_3$ | $6-OCH_3$ | H | $CH_3$ | N | |
| 447 | $CH_3-CH_2$ | H | H | $CH_3$ | CH | Öl (E) |
| 448 | $CH_3-CH_2$ | H | H | $CH_3$ | N | Öl (E) |

| Verb.-Nr. | $R^1$ | $R^2$ | $R^3$ | $R^4$ | X | Fp. (°C) |
|---|---|---|---|---|---|---|
| 449 | $CH_3-CH_2$ | 2-Cl | 5-Cl | $CH_3$ | CH | |
| 450 | $CH_3-CH_2$ | 2-Cl | 5-Cl | $CH_3$ | N | |
| 451 | $CH_3-CH_2$ | 4-Cl | H | $CH_3$ | CH | |
| 452 | $CH_3-CH_2$ | 4-Cl | H | $CH_3$ | N | |
| 453 | $CH_3-CH_2$ | $4-CH_3$ | H | $CH_3$ | CH | |
| 454 | $CH_3-CH_2$ | $4-CH_3$ | H | $CH_3$ | N | |
| 455 | $CH_3-CH_2$ | $5-OCH_3$ | H | $CH_3$ | CH | |
| 456 | $CH_3-CH_2$ | $5-OCH_3$ | H | $CH_3$ | N | |
| 457 | $CH_3-CH_2$ | $6-OCH_3$ | H | $CH_3$ | CH | |
| 458 | $CH_3-CH_2$ | $6-OCH_3$ | H | $CH_3$ | N | |
| 459 | $CH_3-CH_2-CH_2$ | H | H | $CH_3$ | CH | Öl (E) |
| 460 | $CH_3-CH_2-CH_2$ | H | H | $CH_3$ | N | 73 - 74 (E) |
| 461 | $CH_2=CH-CH_2$ | H | H | $CH_3$ | CH | Öl (E) |
| 462 | $CH_2=CH-CH_2$ | H | H | $CH_3$ | N | 51 - 53 (E) |
| 463 | $CH_3-CH(CH_3)$ | H | H | $CH_3$ | CH | Öl (E) |
| 464 | $CH_3-CH(CH_3)$ | H | H | $CH_3$ | N | 58 - 60 (E) |
| 465 | $HC\equiv C-CH_2$ | H | H | $CH_3$ | CH | |
| 466 | $HC\equiv C-CH_2$ | H | H | $CH_3$ | N | |
| 467 | cyclo-$C_3H_5-CH_2$ | H | H | $CH_3$ | CH | |
| 468 | cyclo-$C_3H_5-CH_2$ | H | H | $CH_3$ | N | |
| 469 | $CH_3-CH_2-CH_2-CH_2$ | H | H | $CH_3$ | CH | Öl (E) |
| 470 | $CH_3-CH_2-CH_2-CH_2$ | H | H | $CH_3$ | N | Öl (E) |

| Verb.-Nr. | $R^1$ | $R^2$ | $R^3$ | $R^4$ | X | Fp.(°C) |
|---|---|---|---|---|---|---|
| 471 | $CH_3-CH=CH-CH_2$ | H | H | $CH_3$ | CH | Öl (E) |
| 472 | $CH_3-CH=CH-CH_2$ | H | H | $CH_3$ | N | 76 - 78 (E) |
| 473 | $CH_3-(CH_2)_5$ | H | H | $CH_3$ | CH | Öl (E) |
| 474 | $CH_3-(CH_2)_5$ | H | H | $CH_3$ | N | Öl (E) |
| 475 | cyclo-$C_6H_{11}$ | H | H | $CH_3$ | CH | |
| 476 | cyclo-$C_6H_{11}$ | H | H | $CH_3$ | N | |
| 477 | $C_6H_5-CH_2$ | H | H | $CH_3$ | CH | |
| 478 | $C_6H_5-CH_2$ | H | H | $CH_3$ | N | Öl (E) |
| 479 | 4-Cl-$C_6H_4-CH_2$ | H | H | $CH_3$ | CH | |
| 480 | 4-Cl-$C_6H_4-CH_2$ | H | H | $CH_3$ | N | |
| 481 | 3-$CF_3-C_6H_4-CH_2$ | H | H | $CH_3$ | CH | |
| 482 | 3-$CF_3-C_6H_4-CH_2$ | H | H | $CH_3$ | N | |
| 483 | 4-Cl-$C_6H_4-CH_2-CH_2$ | H | H | $CH_3$ | CH | |
| 484 | 4-Cl-$C_6H_4-CH_2-CH_2$ | H | H | $CH_3$ | N | |
| 485 | $C_6H_5-CH_2-CH_2-CH_2$ | H | H | $CH_3$ | CH | |
| 486 | $C_6H_5-CH_2-CH_2-CH_2$ | H | H | $CH_3$ | N | |
| 487 | $C_6H_5-(CH_2)_4$ | H | H | $CH_3$ | CH | |
| 488 | $C_6H_5-(CH_2)_4$ | H | H | $CH_3$ | N | |
| 489 | $C_6H_5-CH_2-CH=CH-CH_2$ | H | H | $CH_3$ | CH | |
| 490 | $C_6H_5-CH_2-CH=CH-CH_2$ | H | H | $CH_3$ | N | |
| 491 | 4-F-$C_6H_4-CH=CH-CH_2-CH_2$ | H | H | $CH_3$ | CH | |
| 492 | 4-F-$C_6H_4-CH=CH-CH_2-CH_2$ | H | H | $CH_3$ | N | |

| Verb.-Nr. | $R^1$ | $R^2$ | $R^3$ | $R^4$ | X | Fp. (°C) |
|---|---|---|---|---|---|---|
| 493 | $t\text{-}C_4H_9O\text{-}CO\text{-}CH_2$ | H | H | $CH_3$ | CH | |
| 494 | $t\text{-}C_4H_9O\text{-}CO\text{-}CH_2$ | H | H | $CH_3$ | N | |
| 495 | $t\text{-}C_4H_9O\text{-}CO\text{-}(CH_2)_3$ | H | H | $CH_3$ | CH | |
| 496 | $t\text{-}C_4H_9O\text{-}CO\text{-}(CH_2)_3$ | H | H | $CH_3$ | N | |
| 497 | $Cl\text{-}CH{=}CH\text{-}CH_2$ | H | H | $CH_3$ | CH | öl (E) |
| 498 | $Cl\text{-}CH{=}CH\text{-}CH_2$ | H | H | $CH_3$ | N | öl (E) |
| 499 | $C_2H_5$ | $6\text{-}OC_2H_5$ | H | $CH_3$ | CH | |
| 500 | $C_2H_5$ | $6\text{-}OC_2H_5$ | H | $CH_3$ | N | |
| 501 | $CH_3\text{-}C(CH_3)_2$ | H | H | $CH_3$ | CH | öl (E) |
| 502 | $CH_3\text{-}C(CH_3)_2$ | H | H | $CH_3$ | N | 83 - 85 (E) |
| 503 | $CH_3\text{-}CH(CH_3)\text{-}CH_2$ | H | H | $CH_3$ | CH | öl (E) |
| 504 | $CH_3\text{-}CH(CH_3)\text{-}CH_2$ | H | H | $CH_3$ | N | 70 - 72 (E) |
| 505 | $CH_2{=}C(CH_3)\text{-}CH_2$ | H | H | $CH_3$ | CH | öl (E) |
| 506 | $CH_2{=}C(CH_3)\text{-}CH_2$ | H | H | $CH_3$ | N | 64 - 65 (E) |
| 507 | $CH_3\text{-}CH(CH_3)\text{-}CH_2\text{-}CH_2$ | H | H | $CH_3$ | CH | öl (E) |
| 508 | $CH_3\text{-}CH(CH_3)\text{-}CH_2\text{-}CH_2$ | H | H | $CH_3$ | N | öl (E) |
| 509 | $CH_3\text{-}(CH_2)_4$ | H | H | $CH_3$ | CH | |
| 510 | $CH_3\text{-}(CH_2)_4$ | H | H | $CH_3$ | N | |
| 511 | $2\text{-}F\text{-}C_6H_4\text{-}CH_2$ | H | H | $CH_3$ | CH | |
| 512 | $2\text{-}F\text{-}C_6H_4\text{-}CH_2$ | H | H | $CH_3$ | N | |
| 513 | $3\text{-}F\text{-}C_6H_4\text{-}CH_2$ | H | H | $CH_3$ | CH | |
| 514 | $3\text{-}F\text{-}C_6H_4\text{-}CH_2$ | H | H | $CH_3$ | N | |

| Verb.-Nr. | $R^1$ | $R^2$ | $R^3$ | $R^4$ | X | Fp. (°C) |
|---|---|---|---|---|---|---|
| 515 | $2-Cl-C_6H_4-CH_2$ | H | H | $CH_3$ | CH | |
| 516 | $2-Cl-C_6H_4-CH_2$ | H | H | $CH_3$ | N | |
| 517 | $3,4-Cl_2-C_6H_3-CH_2$ | H | H | $CH_3$ | CH | |
| 518 | $3,4-Cl_2-C_6H_3-CH_2$ | H | H | $CH_3$ | N | |
| 519 | $2,6-Cl_2-C_6H_3-CH_2$ | H | H | $CH_3$ | CH | |
| 520 | $2,6-Cl_2-C_6H_3-CH_2$ | H | H | $CH_3$ | N | |
| 521 | $C_6H_5-CH_2-CH_2$ | H | H | $CH_3$ | CH | |
| 522 | $C_6H_5-CH_2-CH_2$ | H | H | $CH_3$ | N | |
| 523 | $C_6H_5-CH=CH-CH_2-CH_2$ | H | H | $CH_3$ | CH | |
| 524 | $C_6H_5-CH=CH-CH_2-CH_2$ | H | H | $CH_3$ | N | |
| 525 | $4-Cl-C_6H_4-CH_2-CH=CH-CH_2$ | H | H | $CH_3$ | CH | |
| 526 | $4-Cl-C_6H_4-CH_2-CH=CH-CH_2$ | H | H | $CH_3$ | N | |
| 527 | $4-CF_3-C_6H_4-CH_2-CH=CH-CH_2$ | H | H | $CH_3$ | CH | |
| 528 | $4-CF_3-C_6H_4-CH_2-CH=CH-CH_2$ | H | H | $CH_3$ | N | |
| 529 | $CH_3$ | H | H | $C_6H_5$ | CH | |
| 530 | $CH_3$ | H | H | $C_6H_5$ | N | |
| 531 | $C_2H_5$ | H | H | $C_6H_5$ | CH | |
| 532 | $C_2H_5$ | H | H | $C_6H_5$ | N | |
| 533 | $CH_3-CH_2-CH_2$ | H | H | $C_6H_5$ | CH | |
| 534 | $CH_3-CH_2-CH_2$ | H | H | $C_6H_5$ | N | |
| 535 | $CH_3-(CH_2)_5$ | H | H | $C_6H_5$ | CH | |
| 536 | $CH_3-(CH_2)_5$ | H | H | $C_6H_5$ | N | |
| 537 | $C_6H_5-CH_2$ | H | H | $C_6H_5$ | CH | |
| 538 | $C_6H_5-CH_2$ | H | H | $C_6H_5$ | N | |

Tabelle 3

Verbindungen der allgemeinen Formel Ic (4-Stellung des Oximethers). Die Konfigurationsangabe bezieht sich auf die β-Methoxy-acrylsäuremethylestergruppe bzw. auf die Glyoxylsäuremethylester-O-methyloximgruppe.

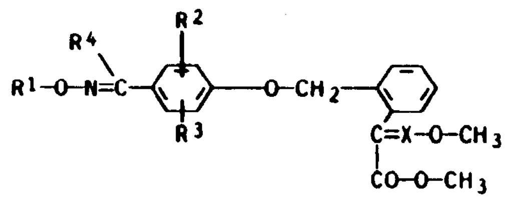

Ic

| Verb.-Nr. | $R^1$ | $R^2$ | $R^3$ | $R^4$ | X | Fp.($^oC$) |
|---|---|---|---|---|---|---|
| 149 | $CH_3$- | H | H | H | CH | 84-86 (E) |
| 150 | $CH_3$- | H | H | H | N | 88-91 (E) |
| 151 | $CH_3$- | 2-Cl | H | H | CH | |
| 152 | $CH_3$- | 2-Cl | H | H | N | |
| 153 | $CH_3$- | 2-$CH_3$ | H | H | CH | |
| 154 | $CH_3$- | 2-$CH_3$ | H | H | N | |
| 155 | $CH_3$- | 2-$OCH_3$ | H | H | CH | Öl (E) |
| 156 | $CH_3$- | 2-$OCH_3$ | H | H | N | 105-107 (E) |
| 157 | $CH_3$- | 3-Cl | H | H | CH | |
| 158 | $CH_3$- | 3-Cl | H | H | N | |
| 159 | $CH_3$- | 3-$CH_3$ | H | H | CH | |
| 160 | $CH_3$- | 3-$CH_3$ | H | H | N | |
| 161 | $CH_3$ | 3-$OCH_3$ | H | H | CH | |
| 162 | $CH_3$- | 3-$OCH_3$ | H | H | N | |

| Verb.-Nr. | $R^1$ | $R^2$ | $R^3$ | $R^4$ | X | Fp. (°C) |
|---|---|---|---|---|---|---|
| 163 | $CH_3$ | 2-Cl | 6-Cl | H | CH | |
| 164 | $CH_3$ | 2-Cl | 6-Cl | H | N | |
| 165 | $CH_3-CH_2-$ | H | H | H | CH | 108-110 (E) |
| 166 | $CH_3-CH_2$ | H | H | H | N | 106-108 (E) |
| 167 | $CH_3-CH_2-$ | 2-Cl | H | H | CH | |
| 168 | $CH_3-CH_2-$ | 2-Cl | H | H | N | |
| 169 | $CH_3-CH_2-$ | $2-CH_3$ | H | H | CH | |
| 170 | $CH_3-CH_2-$ | $2-CH_3$ | H | H | N | |
| 171 | $CH_3-CH_2-$ | $2-OCH_3$ | H | H | CH | |
| 172 | $CH_3-CH_2-$ | $2-OCH_3$ | H | H | N | |
| 173 | $CH_3-CH_2-$ | 3-Cl | H | H | CH | |
| 174 | $CH_3-CH_2-$ | 3-Cl | H | H | N | |
| 175 | $CH_3-CH_2-$ | $3-CH_3$ | H | H | CH | |
| 176 | $CH_3-CH_2-$ | $3-CH_3$ | H | H | N | |
| 177 | $CH_3-CH_2-$ | $3-OCH_3$ | H | H | CH | |
| 178 | $CH_3-CH_2-$ | $3-OCH_3$ | H | H | N | |
| 179 | $CH_3-CH_2-$ | 2-Cl | 6-Cl | H | CH | |
| 180 | $CH_3-CH_2-$ | 2-Cl | 6-Cl | H | N | |
| 181 | $CH_3-CH_2-CH_2-$ | H | H | H | CH | |
| 182 | $CH_3-CH_2-CH_2-$ | H | H | H | N | |
| 183 | $CH_2=CH-CH_2-$ | H | H | H | CH | 103-105 (E) |
| 184 | $CH_2=CH-CH_2-$ | H | H | H | N | 82- 84 (E) |
| 185 | $CH_3-CH(CH_3)-$ | H | H | H | CH | |
| 186 | $CH_3-CH(CH_3)-$ | H | H | H | N | |
| 187 | $HC\equiv C-CH_2-$ | H | H | H | CH | |

| Verb.-Nr. | $R^1$ | $R^2$ | $R^3$ | $R^4$ | X | Fp.(°C) |
|---|---|---|---|---|---|---|
| 188 | $HC{\equiv}C-CH_2-$ | H | H | H | N | |
| 189 | cyclo-$C_3H_5-CH_2-$ | H | H | H | CH | |
| 190 | cyclo-$C_3H_5-CH_2-$ | H | H | H | N | |
| 191 | $CH_3-CH_2-CH_2-CH_2-$ | H | H | H | CH | |
| 192 | $CH_3-CH_2-CH_2-CH_2-$ | H | H | H | N | |
| 193 | $CH_3-CH{=}CH-CH_2-$ | H | H | H | CH | |
| 194 | $CH_3-CH{=}CH-CH_2-$ | H | H | H | N | |
| 195 | $CH_3-(CH_2)_5-$ | H | H | H | CH | 62- 63 (E) |
| 196 | $CH_3-(CH_2)_5-$ | H | H | H | N | 72- 73 (E) |
| 197 | cyclo-$C_6H_{11}$ | H | H | H | CH | |
| 198 | cyclo-$C_6H_{11}-$ | H | H | H | N | |
| 199 | $C_6H_5-CH_2-$ | H | H | H | CH | 103-105 (E) |
| 200 | $C_6H_5-CH_2-$ | H | H | H | N | 151-153 (E) |
| 201 | $4-Cl-C_6H_4-CH_2-$ | H | H | H | CH | |
| 202 | $4-Cl-C_6H_4-CH_2-$ | H | H | H | N | |
| 203 | $3-CF_3-C_6H_4-CH_2-$ | H | H | H | CH | |
| 204 | $3-CF_3-C_6H_4-CH_2-$ | H | H | H | N | |
| 205 | $4-Cl-C_6H_4-CH_2-CH_2-$ | H | H | H | CH | |
| 206 | $4-Cl-C_6H_4-CH_2-CH_2-$ | H | H | H | N | |
| 207 | $C_6H_5-CH_2-CH_2-CH_2-$ | H | H | H | CH | |
| 208 | $C_6H_5-CH_2-CH_2-CH_2-$ | H | H | H | N | |
| 209 | $C_6H_5-(CH_2)_4-$ | H | H | H | CH | |

| Verb.-Nr. | $R^1$ | $R^2$ | $R^3$ | $R^4$ | X | Fp. (°C) |
|---|---|---|---|---|---|---|
| 210 | $C_6H_5-(CH_2)_4-$ | H | H | H | N | |
| 211 | $C_6H_5-CH_2-CH=CH-CH_2-$ | H | H | H | CH | |
| 212 | $C_6H_5-CH_2-CH=CH-CH_2-$ | H | H | H | N | |
| 213 | $4-F-C_6H_4-CH=CH-CH_2-CH_2-$ | H | H | H | CH | |
| 214 | $4-F-C_6H_4-CH=CH-CH_2-CH_2-$ | H | H | H | N | |
| 215 | $t-C_4H_9O-CO-CH_2-$ | H | H | H | CH | |
| 216 | $t-C_4H_9O-CO-CH_2-$ | H | H | H | N | |
| 217 | $t-C_4H_9O-CO-(CH_2)_3-$ | H | H | H | CH | |
| 218 | $t-C_4H_9O-CO-(CH_2)_3-$ | H | H | H | N | |
| 219 | $Cl-CH=CH-CH_2-$ | H | H | H | CH | Öl (E) |
| 220 | $Cl-CH=CH-CH_2-$ | H | H | H | N | 95- 97 (E) |
| 281 | $C_2H_5$ | $6-OC_2H_5$ | H | H | CH | |
| 282 | $C_2H_5$ | $6-OC_2H_5$ | H | H | N | |
| 283 | $CH_3-C(CH_3)_2-$ | H | H | H | CH | |
| 284 | $CH_3-C(CH_3)_2-$ | H | H | H | N | |
| 285 | $CH_3-CH(CH_3)-CH_2-$ | H | H | H | CH | |
| 286 | $CH_3-CH(CH_3)-CH_2-$ | H | H | H | N | |
| 287 | $CH_2=C(CH_3)-CH_2$ | H | H | H | CH | 100-102 (E) |
| 288 | $CH_2=C(CH_3)-CH_2$ | H | H | H | N | 95- 96 (E) |
| 289 | $CH_3-CH(CH_3)-CH_2-CH_2-$ | H | H | H | CH | |
| 290 | $CH_3-CH(CH_3)-CH_2-CH_2-$ | H | H | H | N | |
| 291 | $CH_3-(CH_2)_4-$ | H | H | H | CH | |
| 292 | $CH_3-(CH_2)_4-$ | H | H | H | N | Öl (E) |
| 293 | $2-F-C_6H_4-CH_2-$ | H | H | H | CH | |

| Verb.-Nr. | $R^1$ | $R^2$ | $R^3$ | $R^4$ | X | Fp. (°C) |
|---|---|---|---|---|---|---|
| 294 | $2\text{-F-}C_6H_4\text{-}CH_2\text{-}$ | H | H | H | N | |
| 295 | $3\text{-F-}C_6H_4\text{-}CH_2\text{-}$ | H | H | H | CH | |
| 296 | $3\text{-F-}C_6H_4\text{-}CH_2\text{-}$ | H | H | H | N | |
| 297 | $2\text{-Cl-}C_6H_4\text{-}CH_2\text{-}$ | H | H | H | CH | |
| 298 | $2\text{-Cl-}C_6H_4\text{-}CH_2\text{-}$ | H | H | H | N | |
| 299 | $3,4\text{-}Cl_2\text{-}C_6H_3\text{-}CH_2\text{-}$ | H | H | H | CH | |
| 300 | $3,4\text{-}Cl_2\text{-}C_6H_3\text{-}CH_2\text{-}$ | H | H | H | N | |
| 301 | $2,6\text{-}Cl_2\text{-}C_6H_3\text{-}CH_2\text{-}$ | H | H | H | CH | |
| 302 | $2,6\text{-}Cl_2\text{-}C_6H_3\text{-}CH_2\text{-}$ | H | H | H | N | |
| 303 | $C_6H_5\text{-}CH_2\text{-}CH_2\text{-}$ | H | H | H | CH | |
| 304 | $C_6H_5\text{-}CH_2\text{-}CH_2\text{-}$ | H | H | H | N | |
| 305 | $C_6H_5\text{-}CH=CH\text{-}CH_2\text{-}CH_2\text{-}$ | H | H | H | CH | |
| 306 | $C_6H_5\text{-}CH=CH\text{-}CH_2\text{-}CH_2\text{-}$ | H | H | H | N | |
| 307 | $4\text{-Cl-}C_6H_4\text{-}CH_2\text{-}CH=CH\text{-}CH_2\text{-}$ | H | H | H | CH | |
| 308 | $4\text{-Cl-}C_6H_4\text{-}CH_2\text{-}CH=CH\text{-}CH_2\text{-}$ | H | H | H | N | |
| 309 | $4\text{-}CF_3\text{-}C_6H_4\text{-}CH_2\text{-}CH=CH\text{-}CH_2\text{-}$ | H | H | H | CH | |
| 310 | $4\text{-}CF_3\text{-}C_6H_4\text{-}CH_2\text{-}CH=CH\text{-}CH_2\text{-}$ | H | H | H | N | |
| 539 | $CH_3$ | H | H | $CH_3$ | CH | Öl (E) |
| 540 | $CH_3$ | H | H | $CH_3$ | N | 99 - 100 (E) |
| 541 | $CH_3$ | 2-Cl | H | $CH_3$ | CH | |
| 542 | $CH_3$ | 2-Cl | H | $CH_3$ | N | |
| 543 | $CH_3$ | $2\text{-}CH_3$ | H | $CH_3$ | CH | |
| 544 | $CH_3$ | $2\text{-}CH_3$ | H | $CH_3$ | N | |
| 545 | $CH_3$ | $2\text{-}OCH_3$ | H | $CH_3$ | CH | |

| Verb.-Nr. | $R^1$ | $R^2$ | $R^3$ | $R^4$ | X | Fp.(°C) |
|---|---|---|---|---|---|---|
| 546 | $CH_3$ | 2-$OCH_3$ | H | $CH_3$ | N | |
| 547 | $CH_3$ | 3-Cl | H | $CH_3$ | CH | |
| 548 | $CH_3$ | 3-Cl | H | $CH_3$ | N | |
| 549 | $CH_3$ | 3-$CH_3$ | H | $CH_3$ | CH | |
| 550 | $CH_3$ | 3-$CH_3$ | H | $CH_3$ | N | |
| 551 | $CH_3$ | 3-$OCH_3$ | H | $CH_3$ | CH | |
| 552 | $CH_3$ | 3-$OCH_3$ | H | $CH_3$ | N | |
| 553 | $CH_3$ | 2-Cl | 6-Cl | $CH_3$ | CH | |
| 554 | $CH_3$ | 2-Cl | 6-Cl | $CH_3$ | N | |
| 555 | $CH_3$-$CH_2$ | H | H | $CH_3$ | CH | 71 - 73 (E) |
| 556 | $CH_3$-$CH_2$ | H | H | $CH_3$ | N | 79 - 80 (E) |
| 557 | $CH_3$-$CH_2$ | 2-Cl | H | $CH_3$ | CH | |
| 558 | $CH_3$-$CH_2$ | 2-Cl | H | $CH_3$ | N | |
| 559 | $CH_3$-$CH_2$ | 2-$CH_3$ | H | $CH_3$ | CH | |
| 560 | $CH_3$-$CH_2$ | 2-$CH_3$ | H | $CH_3$ | N | |
| 561 | $CH_3$-$CH_2$ | 2-$OCH_3$ | H | $CH_3$ | CH | |
| 562 | $CH_3$-$CH_2$ | 2-$OCH_3$ | H | $CH_3$ | N | |
| 563 | $CH_3$-$CH_2$ | 3-Cl | H | $CH_3$ | CH | |
| 564 | $CH_3$-$CH_2$ | 3-Cl | H | $CH_3$ | N | |
| 565 | $CH_3$-$CH_2$ | 3-$CH_3$ | H | $CH_3$ | CH | |
| 566 | $CH_3$-$CH_2$ | 3-$CH_3$ | H | $CH_3$ | N | |
| 567 | $CH_3$-$CH_2$ | 3-$OCH_3$ | H | $CH_3$ | CH | |
| 568 | $CH_3$-$CH_2$ | 3-$OCH_3$ | H | $CH_3$ | N | |
| 569 | $CH_3$-$CH_2$ | 2-Cl | 6-Cl | $CH_3$ | CH | |

| Verb.-Nr. | $R^1$ | $R^2$ | $R^3$ | $R^4$ | X | Fp. (°C) |
|---|---|---|---|---|---|---|
| 570 | $CH_3-CH_2$ | 2-Cl | 6-Cl | $CH_3$ | N | |
| 571 | $CH_3-CH_2-CH_2$ | H | H | $CH_3$ | CH | 97 - 99 (E) |
| 572 | $CH_3-CH_2-CH_2$ | H | H | $CH_3$ | N | 100 - 101 (E) |
| 573 | $CH_2=CH-CH_2$ | H | H | $CH_3$ | CH | 90 - 92 (E) |
| 574 | $CH_2=CH-CH_2$ | H | H | $CH_3$ | N | 107 - 108 (E) |
| 575 | $CH_3-CH(CH_3)$ | H | H | $CH_3$ | CH | 120 - 123 (E) |
| 576 | $CH_3-CH(CH_3)$ | H | H | $CH_3$ | N | 109 - 110 (E) |
| 577 | $HC=C-CH_2$ | H | H | $CH_3$ | CH | |
| 578 | $HC=C-CH_2$ | H | H | $CH_3$ | N | |
| 579 | cyclo-$C_3H_5-CH_2$ | H | H | $CH_3$ | CH | |
| 580 | cyclo-$C_3H_5-CH_2$ | H | H | $CH_3$ | N | |
| 581 | $CH_3-CH_2-CH_2-CH_2$ | H | H | $CH_3$ | CH | 64 - 66 (E) |
| 582 | $CH_3-CH_2-CH_2-CH_2$ | H | H | $CH_3$ | N | Öl (E) |
| 583 | $CH_3-CH=CH-CH_2$ | H | H | $CH_3$ | CH | Öl (E) |
| 584 | $CH_3-CH=CH-CH_2$ | H | H | $CH_3$ | N | 100 - 103 (E) |
| 585 | $CH_3-(CH_2)_5$ | H | H | $CH_3$ | CH | 65 - 67 (E) |
| 586 | $CH_3-(CH_2)_5$ | H | H | $CH_3$ | N | 60 - 63 (E) |
| 587 | cyclo-$C_6H_{11}$ | H | H | $CH_3$ | CH | |
| 588 | cyclo-$C_6H_{11}$ | H | H | $CH_3$ | N | |
| 589 | $C_6H_5-CH_2$ | H | H | $CH_3$ | CH | 110 - 112 (E) |
| 590 | $C_6H_5-CH_2$ | H | H | $CH_3$ | N | 104 - 106 (E) |
| 591 | 4-Cl-$C_6H_4-CH_2$ | H | H | $CH_3$ | CH | |
| 592 | 4-Cl-$C_6H_4-CH_2$ | H | H | $CH_3$ | N | |
| 593 | 3-$CF_3-C_6H_4-CH_2$ | H | H | $CH_3$ | CH | |

| Verb.-Nr. | $R^1$ | $R^2$ | $R^3$ | $R^4$ | X | Fp. (°C) |
|---|---|---|---|---|---|---|
| 594 | $3-CF_3-C_6H_4-CH_2$ | H | H | $CH_3$ | N | |
| 595 | $4-Cl-C_6H_4-CH_2-CH_2$ | H | H | $CH_3$ | CH | |
| 596 | $4-Cl-C_6H_4-CH_2-CH_2$ | H | H | $CH_3$ | N | |
| 597 | $C_6H_5-CH_2-CH_2-CH_2$ | H | H | $CH_3$ | CH | |
| 598 | $C_6H_5-CH_2-CH_2-CH_2$ | H | H | $CH_3$ | N | |
| 599 | $C_6H_5-(CH_2)_4$ | H | H | $CH_3$ | CH | |
| 600 | $C_6H_5-(CH_2)_4$ | H | H | $CH_3$ | N | |
| 601 | $C_6H_5-CH_2-CH=CH-CH_2$ | H | H | $CH_3$ | CH | |
| 602 | $C_6H_5-CH_2-CH=CH-CH_2$ | H | H | $CH_3$ | N | |
| 603 | $4-F-C_6H_4-CH=CH-CH_2-CH_2$ | H | H | $CH_3$ | CH | |
| 604 | $4-F-C_6H_4-CH=CH-CH_2-CH_2$ | H | H | $CH_3$ | N | |
| 605 | $t-C_4H_9O-CO-CH_2$ | H | H | $CH_3$ | CH | |
| 606 | $t-C_4H_9O-CO-CH_2$ | H | H | $CH_3$ | N | |
| 607 | $t-C_4H_9O-CO-(CH_2)_3$ | H | H | $CH_3$ | CH | |
| 608 | $t-C_4H_9O-CO-(CH_2)_3$ | H | H | $CH_3$ | N | |
| 609 | $Cl-CH=CH-CH_2$ | H | H | $CH_3$ | CH | 98 - 100 (E) |
| 610 | $Cl-CH=CH-CH_2$ | H | H | $CH_3$ | N | 105 - 107 (E) |
| 611 | $C_2H_5$ | $6-OC_2H_5$ | H | $CH_3$ | CH | |
| 612 | $C_2H_5$ | $6-OC_2H_5$ | H | $CH_3$ | N | |
| 613 | $CH_3-C(CH_3)_2$ | H | H | $CH_3$ | CH | 88 - 90 (E) |
| 614 | $CH_3-C(CH_3)_2$ | H | H | $CH_3$ | N | 75 - 78 (E) |
| 615 | $CH_3-CH(CH_3)-CH_2$ | H | H | $CH_3$ | CH | 85 - 87 (E) |
| 616 | $CH_3-CH(CH_3)-CH_2$ | H | H | $CH_3$ | N | 79 - 81 (E) |
| 617 | $CH_2=C(CH_3)-CH_2$ | H | H | $CH_3$ | CH | 94 - 96 (E) |

| Verb.-Nr. | $R^1$ | $R^2$ | $R^3$ | $R^4$ | X | Fp.(°C) |
|---|---|---|---|---|---|---|
| 618 | $CH_2=C(CH_3)-CH_2$ | H | H | $CH_3$ | N | 88 - 89 (E) |
| 619 | $CH_3-CH(CH_3)-CH_2-CH_2$ | H | H | $CH_3$ | CH | 46 - 48 (E) |
| 620 | $CH_3-CH(CH_3)-CH_2-CH_2$ | H | H | $CH_3$ | N | Öl (E) |
| 621 | $CH_3-(CH_2)_4$ | H | H | $CH_3$ | CH | |
| 622 | $CH_3-(CH_2)_4$ | H | H | $CH_3$ | N | |
| 623 | $2-F-C_6H_4-CH_2$ | H | H | $CH_3$ | CH | |
| 624 | $2-F-C_6H_4-CH_2$ | H | H | $CH_3$ | N | |
| 625 | $3-F-C_6H_4-CH_2$ | H | H | $CH_3$ | CH | |
| 626 | $3-F-C_6H_4-CH_2$ | H | H | $CH_3$ | N | |
| 627 | $2-Cl-C_6H_4-CH_2$ | H | H | $CH_3$ | CH | |
| 628 | $2-Cl-C_6H_4-CH_2$ | H | H | $CH_3$ | N | |
| 629 | $3,4-Cl_2-C_6H_3-CH_2$ | H | H | $CH_3$ | CH | |
| 630 | $3,4-Cl_2-C_6H_3-CH_2$ | H | H | $CH_3$ | N | |
| 631 | $2,6-Cl_2-C_6H_3-CH_2$ | H | H | $CH_3$ | CH | |
| 632 | $2,6-Cl_2-C_6H_3-CH_2$ | H | H | $CH_3$ | N | |
| 633 | $C_6H_5-CH_2-CH_2$ | H | H | $CH_3$ | CH | |
| 634 | $C_6H_5-CH_2-CH_2$ | H | H | $CH_3$ | N | |
| 635 | $C_6H_5-CH=CH-CH_2-CH_2$ | H | H | $CH_3$ | CH | |
| 636 | $C_6H_5-CH=CH-CH_2-CH_2$ | H | H | $CH_3$ | N | |
| 637 | $4-Cl-C_6H_4-CH_2-CH=CH-CH_2$ | H | H | $CH_3$ | CH | |
| 638 | $4-Cl-C_6H_4-CH_2-CH=CH-CH_2$ | H | H | $CH_3$ | N | |
| 639 | $4-CF_3-C_6H_4-CH_2-CH=CH-CH_2$ | H | H | $CH_3$ | CH | |
| 640 | $4-CF_3-C_6H_4-CH_2-CH=CH-CH_2$ | H | H | $CH_3$ | N | |
| 641 | $CH_3$ | H | H | $C_6H_5$ | CH | |

| Verb.-Nr. | $R^1$ | $R^2$ | $R^3$ | $R^4$ | X | Fp. (°C) |
|---|---|---|---|---|---|---|
| 642 | $CH_3$ | H | H | $C_6H_5$ | N | |
| 643 | $C_2H_5$ | H | H | $C_6H_5$ | CH | |
| 644 | $C_2H_5$ | H | H | $C_6H_5$ | N | |
| 645 | $CH_3-CH_2-CH_2$ | H | H | $C_6H_5$ | CH | |
| 646 | $CH_3-CH_2-CH_2$ | H | H | $C_6H_5$ | N | |
| 647 | $CH_3-(CH_2)_5$ | H | H | $C_6H_5$ | CH | |
| 648 | $CH_3-(CH_2)_5$ | H | H | $C_6H_5$ | N | |
| 649 | $C_6H_5-CH_2$ | H | H | $C_6H_5$ | CH | |
| 650 | $C_6H_5-CH_2$ | H | H | $C_6H_5$ | N | |

Tabelle 4:

$^1$H-NMR-Daten ausgewählter Verbindungen aus den Tabellen 1, 2 und 3. Die chemische Verschiebung (δ) wird in ppm relativ zu Tetramethylsilan angegeben. Als Lösungsmittel dient $CDCl_3$.

Verbindung Nr. 1

3.67 (s, 3H); 3.77 (s, 3H); 3.93 (s, 3H); 4.98 (s, 2H); 6.81 - 7.81 (m, 8H); 7.57 (s, 1H); 8.49 (s, 1H).

Verbindung Nr. 2

3.40 (s, 3H); 3.95 (s, 3H); 4.05 (s, 3H); 4.95 (s, 2H); 6.80 - 7.85 (m, 8H); 8.45 (s, 1H).

Verbindung Nr. 23

1.31 (t, 3H); 3.67 (s, 3 H); 3.79 (s, 3H); 4.20 (q, 2H); 4.97 (s, 2H); 6.83 - 7.83 (m, 8H); 7.59 (s, 1H); 8.51 (s, 1H).

Verbindung Nr. 24

1.30 (t, 3H); 3.80 (s, 3H); 4.05 (s, 3H); 4.20 (q, 2H); 4.95(s, 2H); 6.80 - 7.85 (m, 8H); 8.45 (s, 1H).

Verbindung Nr. 85

3.65 (s, 3H); 3.68 (s, 3H); 3.92 (s, 3H); 4.95 (s, 2H); 6.85 - 7.52 (m, 8H); 7.55 (s, 1H); 7.98 (s, 1H).

Verbindung Nr. 97

1.28 (t, 3H); 3.68 (s, 3H); 3.73 (s, 3H); 4.20 (q, 2H); 4.97 (s, 2H); 6.85- 7.53 (m, 8H); 7.57 (s, 1H); 8.00 (s, 1H).

Verbindung Nr. 149

3.68 (s, 3H); 3.77 (s, 3H); 3,92 (s, 3H); 4.98 (s, 2H); 6.87 - 7.53 (m, 8H); 7.59 (s, 1H); 7.98 (s, 1H).

Verbindung Nr. 165

1.31 (t, 3H); 3.69 (s, 3H); 3.80 (s, 3H); 4.20 (q, 2H); 5.00 (s, 2H); 6.87 - 7.53 (m, 8H); 7.60 (s, 1H); 8.00 (s, 1H).

Verbindung Nr. 447

1.32 (t,3H); 2.18 (s,3H); 3.68 (s,3H); 3.77 (s,3H); 4.22 (q,2H); 4.97 (s,2H); 6.83-7.53 (m,8H); 7.55 (5,1H).

Verbindung Nr. 488

1.32 (t,3H); 2.17 (s,3H); 3.82 (s,3H); 4.00 (s,3H); 4.23 (q,4H); 4.97 (s,2H); 6.83-7.57 (m,8H).

Verbindung Nr. 470

0.95 (t,3H); 1.43 (m,2H); 1.70 (m,2H); 2.18 (s,3H); 3.83 (s,3H); 4.00 (s,3H); 4.17 (t,2H); 4.97 (s,2H); 6.82-7.55 (m,8H).

Verbindung Nr. 474

0.87 (t,3H); 1.32 (m,6H); 1.70 (m,2H); 2.18 (s,3H); 3.83 (s,3H); 4.02 (s,3H); 4.17 (t,2H); 4.95 (s,2H); 6.83-7.57 (m,8H).

Verbindung Nr. 478

2.22 (s,3H); 3.78 (s,3H); 4.00 (s,3H); 4.97 (s,2H); 5.23 (s,2H); 6.82-7.53 (m,8H).

Verbindung Nr. 556

1.33 (t,3H); 2.20 (s,3H); 3.83 (s,3H); 4.02 (s,3H); 4.22 (q,2H); 4.97 (s,2H); 6.85-7.60 (m,8H).

Verbindung Nr. 582

0.97 (t,3H); 1.40 (m,2H); 1.68 (m,2H); 2.17 (s,3H); 3.83 (s,3H); 4.00 (s,3H); 4.15 (t,2H); 4.95 (s,2H); 6.82-7.57 (m,8H).

Verbindung Nr. 586

0.88 (t,3H); 1.32 (m,6H); 1.70 (m,2H); 2.18 (s,3H); 3.83 (s,3H); 4.01 (s,3H); 4.15 (t,2H); 4.95 (s,2H); 6.83-7.57 (m,8H).

Verbindung Nr. 590

2.22 (s,3H); 3.83 (s,3H); 4.02 (s,3H), 4.95 (s,2H); 5.22 (s,2H); 6.82-7.57 (m,8H).

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet,

beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 85 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 97 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 447 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 470 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 149 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 165 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 590 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 448 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 85 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 2-(Phenoxymethyl)-phenylglyoxylsäure-methylester-O-methyloxim (A) - bekannt aus EP 253 213 - benutzt.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis zeigt, daß die Wirkstoffe 85 und 97 bei der Anwendung als 0,025 %ige (Gew.%) Spritzbrühe eine bessere fungizide Wirkung zeigen (100 %) als der bekannte Vergleichswirkstoff A (35 %).

In einem anderen Versuch wurde festgestellt, daß die Wirkstoffe 111, 112, 113, 119, 123, 195, 220, 262, 265, 266, 273, 275, 447 und 470 bei der Anwendung als 0,025 %ige (Gew.%) Spritzbrühe eine bessere fungizide Wirkung zeigen (95 %) als der bekannte Vergleichswirkstoff A (35 %).

Anwendungsbeispiel 2

Wirksamkeit gegen Plasmopara viticola

Blätter von Topfreben der Sorte "Müller Thurgau" wurden mit wäßriger Spritzbrühe, die 80% Wirkstoff und 20% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 85, 97, 149 und 165 bei der Anwendung als 0,0125%ige Spritzbrühe eine bessere fungizide Wirkung zeigen (100%) als der bekannte Vergleichswirkstoff A (50%).

In einem anderen Versuch wurde festgestellt, daß die Wirkstoffe 86, 98, 111, 113, 114, 119, 120, 123, 124, 127, 150, 155, 183, 184, 199, 219, 220, 263, 265, 267, 273, 275, 277, 287, 288, 447 und 448 bei der Anwendung als 0,0125 %ige Spritzbrühe eine bessere fungizide Wirkung zeigen (95 %) als der bekannte Vergleichswirkstoff A (50 %).

## Patentansprüche

1. Substituierte Oximether der allgemeinen Formel I

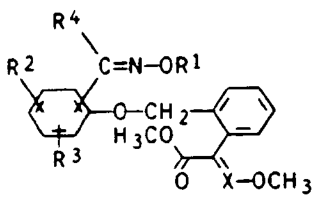

I

in der

$R^1$

$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkyl, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,

$R^2$ und $R^3$

gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,

$R^4$

Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und

X CH oder N bedeutet.

2. Verbindung der Formel I gemäß Anspruch 1, in der $R^1$ Ethyl, $R^2$ und $R^3$ Wasserstoff, $R^4$ Methyl bedeuten und X die Bedeutung CH hat, wobei der Oximetherrest in 3-Stellung am Phenylrest steht.

3. Verbindung der Formel I gemäß Anspruch 1, in der $R^1$ Ethyl, $R^2$ und $R^3$ Wasserstoff, $R^4$ Methyl bedeuten

und X die Bedeutung Stickstoff hat, wobei der Oximetherrest -C($R^4$)=N-O$R^1$ in 3-Stellung am Phenylrest steht.

4. Verbindung der Formel I gemäß Anspruch 1, in der $R^1$ Methyl, $R^2$, $R^3$ und $R^4$ Wasserstoff bedeuten und X die Bedeutung CH hat, wobei der Oximetherrest in 3-Stellung am Phenylrest steht.

5. Verbindung der Formel I gemäß Anspruch 1, in der $R^1$ Methyl, $R^2$, $R^3$ und $R^4$ Wasserstoff bedeuten und X die Bedeutung CH hat, wobei der Oximether in 4-Stellung am Phenylrest steht.

6. Verfahren zur Herstellung substituierter Oximether der allgemeinen Formel I

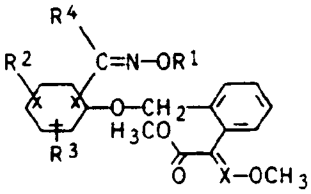

I

in der
$R^1$
$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,
$R^2$ und $R^3$
gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,
$R^4$
Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und
X CH oder N bedeutet,
dadurch gekennzeichnet, daß man einen substituierten Oximether der allgemeinen Formel II ($R^1$ ,$R^2$, $R^3$ und $R^4$ haben die oben angegebene Bedeutung) mit einer substituierten Benzylverbindung der allgemeinen Formel III (X hat die oben angegebene Bedeutung), in der Y eine Abgangsgruppe bedeutet, umsetzt.

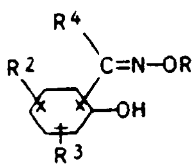

II

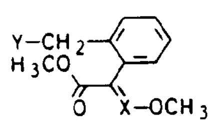

III

7. Verfahren zur Herstellung substituierter Oximether der allgemeinen Formel I

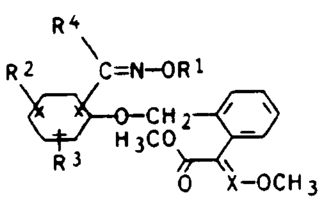

I

in der
$R^1$
$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,
$R^2$ und $R^3$
gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und bedeuten,
$R^4$
Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und
X CH oder N bedeutet,
dadurch gekennzeichnet, daß man eine substituierte Carbonylverbindung der allgemeinen Formel VII ($R^2$, $R^3$, $R^4$ und X haben die oben angegebene Bedeutung) mit einem substutituierten Hydroxylamin der allgemeinen Formel VIII ($R^1$ hat die oben angegebene Bedeutung) oder mit einem Säureadditionssalz der Verbindung VIII umsetzt.

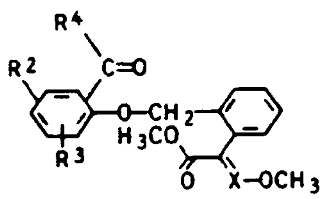

$H_2N-OR^1$

VII VIII

8. Substituierte Carbonylverbindungen der allgemeinen Formel VII,

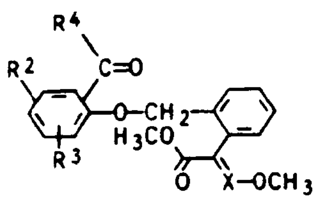

VII

in der
$R^1$
$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,
$R^2$ und $R^3$ gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,
$R^4$
Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und
X CH oder N bedeutet.

9. Fungizid, enthaltend einen inerten Trägerstoff und eine fungizid wirksame Menge eines substituierten Oximethers der allgemeinen Formel I

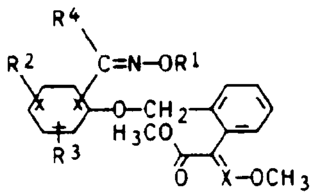

I

in der

$R^1$

$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,

$R^2$ und $R^3$

gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,

$R^4$

Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und

X CH oder N bedeutet.

**10.** Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines substituierten Oximethers der allgemeinen Formel I behandelt,

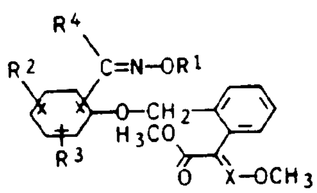

I

in der

$R^1$

$C_1$-$C_6$-Alkyl, $C_3$-$C_6$-Alkenyl, $C_3$-$C_4$-Alkinyl, $C_1$-$C_6$-Halogenalkyl, $C_3$-$C_6$-Halogenalkenyl, $C_1$-$C_4$-Alkoxy-$C_1$-$C_6$-alkyl, $C_3$-$C_6$-Cycloalkly, $C_3$-$C_6$-Cycloalkyl-$C_1$-$C_4$-alkyl, Cyan-$C_1$-$C_6$-alkyl, $C_1$-$C_6$-Alkoxycarbonyl-$C_1$-$C_6$-alkyl, Aryl-$C_1$-$C_6$-alkyl, Heteroaryl-$C_1$-$C_6$-alkyl, Aryl-$C_3$-$C_6$-alkenyl oder Aryloxy-$C_1$-$C_6$-alkyl bedeutet, wobei der aromatische oder heteroaromatische Ring gegebenenfalls durch einen oder mehrere der folgenden Reste substituiert ist: $C_1$-$C_4$-Alkyl, $C_1$-$C_2$-Halogenalkyl, $C_3$-$C_6$-Cycloalkyl, $C_1$-$C_4$-Alkoxy, $C_1$-$C_2$-Halogenalkoxy, Halogen, Aryl, Aryloxy,

$R^2$ und $R^3$

gleich oder verschieden sind und Wasserstoff, $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro bedeuten,

$R^4$

Wasserstoff, $C_1$-$C_6$-Alkyl oder Aryl bedeutet, wobei der aromatische Ring gegebenenfalls durch einen oder mehrere der folgenden Reste substituiert ist: $C_1$-$C_4$-Alkyl, $C_1$-$C_2$-Halogenalkyl, $C_1$-$C_4$-Alkoxy, $C_1$-$C_2$-Halogenalkoxy, Halogen, Cyano oder Nitro und

X CH oder N bedeutet.

## Claims

**1.** A substituted oxime ether of the general formula I

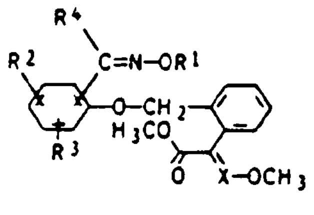

I,

where $R^1$ is $C_1$-$C_6$-alkyl, $C_3$-$C_6$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl-$C_1$-$C_6$-alkyl, aryl-$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,
$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,
$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and
X is CH or N.

2. A compound of the formula I as claimed in claim 1, where $R^1$ is ethyl, $R^2$ and $R^3$ are hydrogen, $R^4$ is methyl and X is CH, the oxime ether residue being in the 3 position on the phenyl radical.

3. A compound of the formula I as claimed in claim 1, where $R^1$ is ethyl, $R^2$ and $R^3$ are hydrogen, $R^4$ is methyl and X is nitrogen, the oxime ether residue -C($R^4$)=N-O$R^1$ being in the 3 position on the phenyl radical.

4. A compound of the formula I as claimed in claim 1, where $R^1$ is methyl, $R^2$, $R^3$ and $R^4$ are hydrogen and X is CH, the oxime ether residue being in the 3 position on the phenyl radical.

5. A compound of the formula I as claimed in claim 1, where $R^1$ is methyl, $R^2$, $R^3$ and $R^4$ are hydrogen and X is CH, the oxime ether being in the 4 position on the phenyl radical.

6. A process for preparing a substituted oxime ether of the general formula I

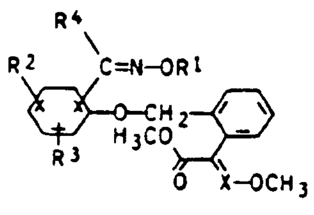

I,

where $R^1$ is $C_1$-$C_6$-alkyl, $C_3$-$C_6$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl-$C_1$-$C_6$-alkyl, aryl-$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,
$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,
$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and
X is CH or N, which comprises reacting a substituted oxime ether of the general formula II ($R^1$, $R^2$, $R^3$ and $R^4$ have the meaning stated above) with a substituted benzyl compound of the general formula III (X has the meaning stated above) where Y is a leaving group.

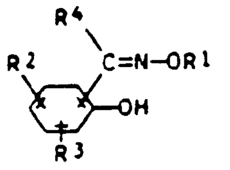

II

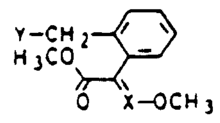

III

7. A process for preparing substituted oxime ethers of the general formula I

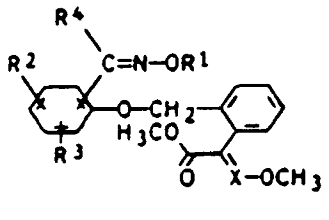

I

where $R^1$ is $C_1$-$C_6$-alkyl, $C_3$-$C_6$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl -$C_1$-$C_6$-alkyl, aryl-$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,
$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,
$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and
X is CH or N, which comprises reacting a substituted carbonyl compound of the general formula VII ($R^2$, $R^3$, $R^4$ and X have the meaning stated above) with a substituted hydroxylamine of the general formula VIII ($R^1$ has the meaning stated above) or with an acid addition salt of the compound VIII.

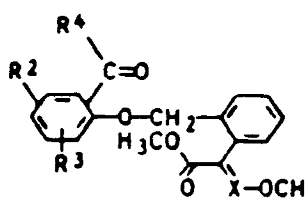

VII

$H_2N-OR^1$

VIII

8. A substituted carbonyl compound of the general formula VII

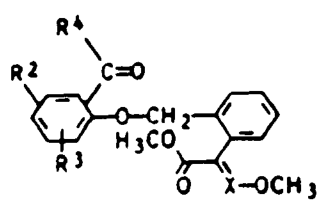

VII

where $R^1$ is $C_1$-$C_6$-alkyl, $C_3$-$C_6$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl-$C_1$-$C_6$-alkyl, aryl-$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,
$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,

$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and

X is CH or N.

9. A fungicide containing an inert carrier and a fungicidally effective amount of a substituted oxime ether of the general formula I

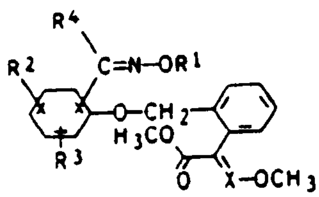

I

where $R^1$ is $C_1$-$C_6$-alkyl, $C_3$-$C_4$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl-$C_1$-$C_6$-alkyl, aryl-$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,

$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,

$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and

X is CH or N.

10. A method for controlling fungi, wherein the fungi, or the materials, plants, seed or the soil threatened by fungus attack are treated with a fungicidally effective amount of a substituted oxime ether of the general formula I

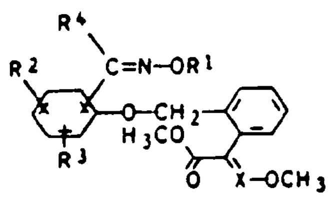

I,

where $R^1$ is $C_1$-$C_6$-alkyl, $C_3$-$C_6$-alkenyl, $C_3$-$C_4$-alkynyl, $C_1$-$C_6$-haloalkyl, $C_3$-$C_6$-haloalkenyl, $C_1$-$C_4$-alkoxy-$C_1$-$C_6$-alkyl, $C_3$-$C_6$-cycloalkyl, $C_3$-$C_6$-cycloalkyl-$C_1$-$C_4$-alkyl, cyano-$C_1$-$C_6$-alkyl, $C_1$-$C_6$-alkoxycarbonyl-$C_1$-$C_6$-alkyl, aryl-$C_1$-$C_6$-alkyl, heteroaryl-$C_1$-$C_6$-alkyl, aryl -$C_3$-$C_6$-alkenyl or aryloxy-$C_1$-$C_6$-alkyl, it being possible for the aromatic or heteroaromatic ring to be substituted by one or more of the following: $C_1$-$C_4$-alkyl, $C_1$-$C_2$-haloalkyl, $C_3$-$C_6$-cycloalkyl, $C_1$-$C_4$-alkoxy, $C_1$-$C_2$-haloalkoxy, halogen, aryl or aryloxy,

$R^2$ and $R^3$ are identical or different and are hydrogen, $C_1$-$C_4$-alkyl, $C_1$-$C_2$-haloalkyl, $C_1$-$C_4$-alkoxy, $C_1$-$C_2$-haloalkoxy, halogen, cyano or nitro,

$R^4$ is hydrogen, $C_1$-$C_6$-alkyl or aryl, it being possible for the aromatic ring to be substituted by one or more of the following: $C_1$-$C_4$-alkyl, $C_1$-$C_2$-haloalkyl, $C_1$-$C_4$-alkoxy, $C_1$-$C_2$-haloalkoxy, halogen, cyano or nitro, and

X is CH or N.

## Revendications

1. Ethers d'oximes substitués de formule générale I

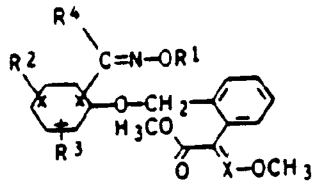

I

dans laquelle
$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$ -alkyle-$C_1$-$_6$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,
$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,
$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et
X = CH ou N.

**2.** Composé de formule I selon la revendication 1, dans laquelle $R^1$ représente éthyle, $R^2$ et $R^3$ hydrogène, $R^4$ méthyle et X est mis pour CH, le reste éther d'oxime étant en position 3 du reste phényle.

**3.** Composé de formule I selon la revendication 1, dans laquelle $R^1$ représente éthyle, $R^2$ et R hydrogène, $R^4$ méthyle et X azote , le reste d'éther d'oxime -C($R^4$)=N-O$R^1$ étant en position 3 du reste phényle.

**4.** Composé de formule I selon la revendication 1, dans laquelle $R^1$ représente méthyle, $R^2$, $R^3$ et $R^4$ hydrogène et X est mis pour le reste d'éther d'oxime étant en position 3 du reste phényle.

**5.** Composé de formule I selon la revendication 1, dans laquelle $R^1$ représente méthyle, $R^2$, $R^3$ et $R^4$ hydrogène et X est mis pour CH, l'éther d'oxime se trouvant en position 4 du reste phényle.

**6.** Procédé de préparation d'éthers d'oximes substitués de formule générale I

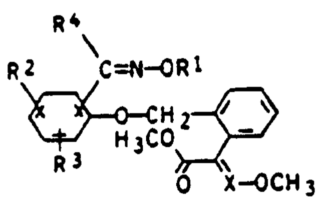

I

dans laquelle
$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$-alkyle-$C_1$-$_6$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,
$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,
$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et
X = CH ou N, caractérisé par le fait que l'on fait réagir un éther d'oxime substitué de formule générale II

($R^1$, $R^2$, $R^3$ et $R^4$ ayant la signification donnée plus haut) avec un composé benzylique substitué de formule générale III (X ayant la signification donnée plus haut), dans laquelle Y représente un groupe éliminable.

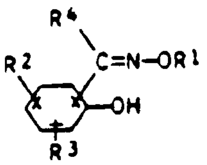

II

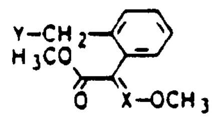

III

**7.** Procédé de préparation d'éthers d'oximes substitués de formule générale I

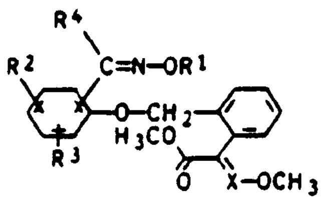

I

dans laquelle

$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$-alkyle-$C_1$-$_6$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,

$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,

$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et

X = CH ou N, caractérisé par le fait que l'on fait réagir un composé carbonyle substitué de formule générale VII ($R^2$, $R^3$, $R^4$ et X ayant la signification donnée plus haut) avec une hydroxylamine substituée de formule générale VIII ($R^1$ ayant la signification donnée plus haut) ou avec un sel d'addition d'acide du composé VIII

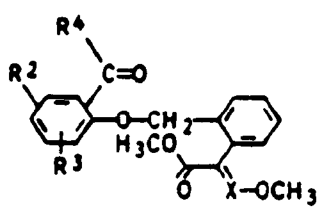

$H_2N-OR^1$

VII VIII

**8.** Composés carbonylés substitués de formule générale VII

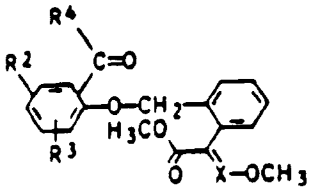

VII

dans laquelle
$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$-alkyle-$C_{1-6}$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,
$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,
$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et
X = CH ou N.

9. Fongicide contenant un véhicule inerte et une quantité efficace comme fongicide d'un éther d'oxime substitué de formule générale I

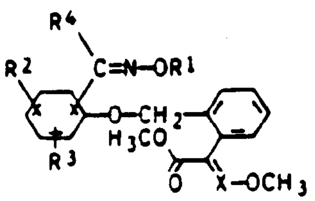

I

dans laquelle
$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$-alkyle-$C_{1-6}$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,
$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,
$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et
X = CH ou N.

10. Procédé de lutte contre les champignons, caractérisé par le fait que l'on traite les champignons ou les matériaux, plantes, semences ou le sol menacés par l'attaque des champignons avec une quantité efficace comme fongicide d'un éther d'oxime substitué, de formule générale I.

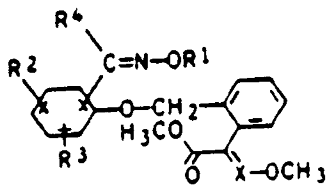

I

dans laquelle

$R^1$ = alkyle en $C_{1-6}$, alcényle en $C_{3-6}$, alcynyle en $C_{3-4}$, halogénalkyle en $C_{1-6}$, halogénalcényle en $C_{3-6}$, alcoxy-$C_1$-$C_4$-alkyle-$C_{1-6}$, cycloalkyle en $C_{3-6}$, cycloalkyl-$C_{3-6}$-alkyle-$C_{1-4}$, cyano-alkyle-$C_{1-6}$, alcoxycarbonyle-$C_{1-6}$-alkyle-$C_{1-6}$, aryl-alkyle-$C_{1-6}$, hétéroaryl-alkyle-$C_{1-6}$, aryl-alcényle-$C_{3-6}$ ou aryloxy-alkyle-$C_{1-6}$, le noyau aromatique ou hétéroaromatique étant éventuellement substitué par un ou plusieurs des restes suivants : alkyle en $C_{1-4}$, halogénalkyle en $C_{1-2}$, cycloylkyle en $C_{3-6}$, alcoxy en $C_{1-4}$, halogénalcoxy en $C_{1-2}$, halogène, aryle, aryloxy,

$R^2$ et $R^3$ sont identiques ou différents et représentent hydrogène, alkyle en $C_{1-4}$, halogénalkyle en $C_{1-2}$, alcoxy en $C_{1-4}$, halogénalcoxy en $C_{1-2}$, halogène, cyano ou nitro,

$R^4$ = hydrogène, alkyle en $C_{1-6}$ ou aryle, le noyau aromatique étant éventuellement substitué par un ou plusieurs des restes suivants : alkyle en $C_{1-4}$, halogénalkyle en $C_{1-2}$, alcoxy en $C_{1-4}$, halogénalcoxy en $C_{1-2}$, halogène, cyano ou nitro et

X = CH ou N.